(19) Europäisches Patentamt / European Patent Office / Office européen des brevets

(11) EP 3 096 792 B1

(12) **EUROPEAN PATENT SPECIFICATION**

(45) Date of publication and mention of the grant of the patent:
**25.09.2019 Bulletin 2019/39**

(21) Application number: **15703743.3**

(22) Date of filing: **23.01.2015**

(51) Int Cl.:
***A61K 31/439*** *(2006.01)* ***A61K 31/566*** *(2006.01)*
***A61K 45/06*** *(2006.01)* ***C07K 16/22*** *(2006.01)*
***A61K 39/395*** *(2006.01)* ***A61K 39/00*** *(2006.01)*
***A61K 31/436*** *(2006.01)* ***A61K 31/5685*** *(2006.01)*

(86) International application number:
**PCT/EP2015/051308**

(87) International publication number:
**WO 2015/110560 (30.07.2015 Gazette 2015/30)**

(54) **CANCER TREATMENT USING AN INSULIN-LIKE GROWTH FACTOR (IGF) RECEPTOR ANTAGONIST IN COMBINATION WITH EXEMESTANE AND EVEROLIMUS**

KREBSBEHANDLUNG MIT EINER INSULINÄHNLICHER WACHSTUMSFAKTOR (IGF) -REZEPTOR ANTAGONIST IN KOMBINATION MIT EXEMESTAN UND EVEROLIMUS

TRAITEMENT DU CANCER AU MOYEN D’UN ANTAGONISTE AU RECEPTEUR DU FACTEUR DE CROISSANCE ANALOGUE À L’INSULINE (IGF) EN COMBINAISON AVEC DE L’EXÉMESTANE ET DE L’EVEROLIMUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2014 EP 14152416**

(43) Date of publication of application:
**30.11.2016 Bulletin 2016/48**

(73) Proprietor: **Boehringer Ingelheim International GmbH**
**55216 Ingelheim am Rhein (DE)**

(72) Inventors:
- **BOGENRIEDER, Thomas**
  **55216 Ingelheim am Rhein (DE)**
- **WEYER-CZERNILOFSKY, Ulrike**
  **55216 Ingelheim am Rhein (DE)**

(74) Representative: **Simon, Elke Anna Maria et al**
**Boehringer Ingelheim GmbH**
**Binger Strasse 173**
**55216 Ingelheim am Rhein (DE)**

(56) References cited:
**WO-A1-2013/169611** **WO-A2-2008/155387**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 096 792 B1

## Description

### Background of the invention

Breast cancer is the most common malignancy in women worldwide. It is estimated that more than 1.6 million new cases of breast cancer occurred globally among women in 2010 (Forouzanfar M, Foreman K, Delossantos AM, Lozano R, Lopez AD, Murray CJL, et al. Lancet 378, 1461 - 1484 (2011). Even though death rates have fallen steadily since 1990, reflecting improvements in early detection and treatment, currently breast cancer is the second leading cause of cancer related death in women. This high death rate reflects the limited effectiveness of current therapeutic options, particularly in patients with advanced disease.

Approximately 75% of primary breast cancers are positive for hormone receptor (HR+). These cancers express Estrogen Receptor (ER) and/ or Progestone Receptor (PgR). Therapies directed at endocrine receptors are important treatment option. For postmenopausal HR+ breast cancer, an aromatase inhibitor (Al), such as letrozole and anastrozole, is the recommended first-line therapy for management. Unfortunately, not all patients have a response to first-line endocrine therapy (primary or de novo resistance), and even patients who have a response will eventually relapse (acquired resistance). On disease progression, second-line treatment options include other classes of aromatase inhibitors (steroidal or nonsteroidal) and the estrogen-receptor (ER) antagonists fulvestrant and tamoxifen (Villarreal-Garza C, Cortes J, Andre F, Verma S. Ann Oncol 23 (10), 2526 - 2535 (2012).

De novo and acquired resistance to endocrine therapy presents a major challenge in the management of HR+ breast cancer. The mammalian target of rapamycin (mTOR) pathway has been shown to play an important role in the resistance to endocrine therapy. Two recently published reports showed that a novel mTOR inhibitor everolimus combined with endocrine therapies were of benefit. In hormone refractory, hormone receptor-positive, HER2-negative metastatic breast cancer patients, tamoxifen plus everolimus resulted in increased clinical benefit compared to tamoxifen alone with improved time to progression (PFS 8.6 vs. 4.5 months) and overall survival (55% reduction in the risk of death associated with combination therapy, HR, 0.45; 95% CI, 0.24 to 0.81; exploratory P=007) (Bachelot T, Bourgier C, Cropet C, Ray-Coquard I, Ferrero JM, Freyer G, et al. J Clin Oncol 30 (22), 2718 - 2724 (2012). In a similar patient population, the Phase III Breast Cancer Trial of Oral Everolimus 2 (BOLERO-2) (Baselga J, Campone M, Piccart M, Burris HA, Rugo HS, Sahmoud T et al. N Engl J Med 2012; 366(6): 520-529) demonstrated that treatment with everolimus plus exemestane more than doubled median progression-free survival to 7.8 months compared with 3.2 months for those treated with exemestane alone (hazard ratio, 0.45; 95% confidence interval, 0.38 - 0.54; one-sided log rank P < .0001) by local investigator assessment. The overall response rate was also improved compared to exemestane alone (12.6 % vs. 1.7%). Based on the result of this trial, everolimus, as the first drug in the mTOR class, was approved by the regulatory agency for the treatment of postmenopausal women with advanced hormone receptor-positive, HER2- negative breast cancer (advanced HR+ BC) in combination with exemstane, after failure of treatment with letrozole or anastrozole.

Insulin-like growth factor-1 (IGF-1; a 70 amino-acid polypeptide) and insulin-like growth factor-2 (IGF-2; a 67 amino-acid polypeptide) are 7.5-kD soluble factors present in serum that can potently stimulate the growth of many mammalian cells (reviewed by Pollack et al., Nature Rev. Can. 4: 505-518, 2004). On secretion into the bloodstream the IGFs form complexes with the IGFBPs which protect them from proteolytic degradation in the serum en route to their target tissues and prevents their association with the IGF receptors. IGFs are also known to be secreted in an autocrine or paracrine manner in target tissues themselves. This is known to occur during normal fetal development where the IGFs play a key role in the growth of tissues, bone and organs. It is also seen in many cancer tissues where there is thought to be paracrine signaling between tumour cells and stromal cells or autocrine IGF production by the tumour cells themselves (reviewed by LeRoith D, Experimental Diab. Res. 4: 205-212, 2003).

IGF-1 and IGF-2 are able to bind to the IGF-1 receptor (IGF-1R) expressed on many normal tissues, which functionally is a 460 kD heterotetramer consisting of a dimerised alpha- and beta-subunit, with similar affinities (Rubin et al., Lab. Invest. 73: 311-31, 1995). IGF-2 can also bind to the IGF-2 receptor, which is thought to prevent IGF-2 from binding and signaling through the IGF-1R. In this respect the IGF-2R has been demonstrated to be a tumour suppressor protein. The IGF-1R is structurally similar to the insulin receptor which exists in two forms, IR-A and IR-B, which differ by an alternatively spliced 12 amino acid exon deletion in the extracellular domain of IR-A. IR-B is the predominant IR isoform expressed in most normal adult tissues where it acts to mediate the effects of insulin on metabolism. IR-A on the other hand is known to be highly expressed in developing fetal tissues but not in adult normal tissues. Recent studies have also shown that IR-A, but not IR-B, is highly expressed in some cancers. The exon deletion in IR-A has no impact on insulin binding but does cause a small conformational change that allows IGF-2 to bind with much higher affinity than for IR-B (Frasca et al., Mol. Cell. Biol. 19: 3278-88, 1999; Pandini et al., J. Biol. Chem. 277: 39684-95, 2002). Thus, because of it's expression in cancer tissues and increased propensity for IGF-2 binding, IR-A may be as important as IGF1-R in mediating the mitogenic effects of IGF-2 in cancer.

Binding of the IGFs to IGF-1R triggers a complex intracellular signaling cascade which results in activation of proteins that stimulate proliferation and survival (reviewed by Pollack et al., Nature Rev. Can. 4: 505-518, 2004).

There is a very large body of scientific, epidemiological and clinical literature implicating a role for the IGFs in the development, progression and metastasis of many different cancer types (reviewed by Jerome et al., End. Rel. Cancer 10: 561-578, 2003; and Pollack et al., Nature Rev. Can. 4: 505-518, 2004).

Preclinical and clinical data indicated that aberrant regulation of the IGF system is attributed to the pathogenesis of breast cancer and also contributes to various stages of breast carcinogenesis. IGF-1R over-expression is common in breast cancer cell lines and fresh tumor biopsies (Cullen KJ, Yee D, Sly WS, Perdue J, Hampton B, Lippman ME, Rosen N Cancer Res 50 (1), 48 - 53 (1990), Yang Y, Yee D. J Mammary Gland Biol Neoplasia 17 (3/4), 251 - 261 (2012), Peyrat JP, Bonneterre J, Beuscart R, Dijane J, Demaille A. Cancer Res 48 (22), 6429 - 6433 (1988), and IGF activity captured in a microarray signature has been associated with poor clinical outcome (Zardavas, D, Basalga J and Piccart M. Nat Rev Clin Oncol. 2013 Apr;10(4):191-210.). Bidirectional crosstalk between the oestrogen and the IGF signalling pathways is well documented (Clarke RB, Howell A, Anderson E. Br J Cancer 75 (2), 251 - 257 (1997), Hamelers IHL, Schaik RFMA van, Teeffelen HAAM van, Sussenbach JS, Steenbergh PH. Exp Cell Res 273, 107 -117) with activation of the latter mediating endocrine resistance (Wiseman LR, Johnson MD, Wakeling AE, Lykkesfeldt AE, May FEB, Westley BR. Eur J Cancer (A) 29 (16), 2256 - 2264 (1993). In ER+ breast cancer resistant to hormonal therapy, InsR-A isoform is the predominant insulin receptor, suggesting an important role for IGF-2 signaling (Bachelot T, Bourgier C, Cropet C, Ray-Coquard I, Ferrero JM, Freyer G, et al. J Clin Oncol 30 (22), 2718 - 2724 (2012). Therefore, the IGF signalling network represents a promising target in advanced breast cancer.

Currently there are three different strategies inhibiting IGF pathways including anti-receptor monoclonal antibodies (ganitumab, cixutumumab, and dalotuzumab), tyrosine kinase inhibitors (TKI, including dual IGF-1R and InsR tyrosine kinase inhibitors BMS-754807, KW2450, and linsitinib), and anti-IGF ligand antibodies (dusigitumab (MEDI-573, Astra Zeneca/MedImmune). These agents are now being tested in the clinic either as monotherapy or in combination with cytotoxic agents and/or other molecularly targeted agents.

The major advantage of neutralizing antibodies for both IGF-1 and IGF-2 is that the sequestration of the ligands ensures that receptor activation by IGF-1 or IGF-2 does not occur, and eliminates the possibility of activation of the InsR-A by IGF-2. Hence it offers a balanced approach with therapeutic potential in a variety of cancers, with few of the pitfalls of targeting the IGF-1R with monoclonal antibodies (mAbs).

A potential mechanism of resistance to mTOR inhibitor therapy is the induction of AKT phosphorylation, which is frequently observed in both preclinical and clinical studies (Sun SY, Rosenberg LM, Wang X, Zhou Z, Yue P, Fu H, et al. Cancer Res. 2005 Aug 15;65(16):7052-8; Tabernero J, Rojo F, Calvo E, Burris H, Judson I, Hazell K, et al. J Clin Oncol 2008; 26(10):1603-1610; Wan X, Harkavy B, Shen N, Grohar P, Helman LJ. Oncogene 26, 1932 - 1940 (2007); O'Reilly KE, Rojo F, She QB, Solit D, Mills GB, Smith D, et al. Cancer Res 66 (3), 1500 - 1508 (2006)). Furthermore, up-regulation of AKT activity is dependent on Insulin-like growth factor (IGF)/Insulin like growth factor type 1 receptor (IGF-1R) signalling. It has been shown in tumors that inhibition of mTOR with rapalogs releases a negative feedback loop on growth factor receptors, including the insulin-like growth factor-1 receptor/insulin receptor substrate (IRS)-1 complex, resulting in activation of IGF-1R signaling and ultimately phosphorylation of AKT, which in turn could potentially counteract the anti-tumor effects of mTOR inhibitors. The activation may be prevented if IGF signaling is blocked simultaneously (Higgins MJ, Baselga J. J Clin Invest 121 (10), 3797 - 3803 (2011).

Combination of an anti-IGF-IR antibody such as dalotuzumab, robatumumab, figitumumab, cixutumumab, ganitumab, Roche RI507 and EM164 with an mTor inhibitor, e.g., everolimus and an aromatase inhibitor, e.g., exemestane for the treatment of breast cancer is disclosed in WO2013/169611. Thus, this combination treatment applies an antibody to the IGF receptor and reports only data on a combination of ridaforolimus (MK-8669), dalotuzumab (MK-0646) and letrozole in an endocrine sensitive, ER+ breast cancer model.

Against this background, the present inventors decided to combine a human anti-IGF antibody with exemestane and everolimus. They have surprisingly found that a triple combination of the human anti-IGF antibody with exemestane and everolimus has a clear advantage in affecting the growth of a breast cancer cell line than the double combination of exemestane and everolimus. Until the present invention it had not been disclosed or contemplated to combine human anti-IGF antibody with exemestane and everolimus to treat patients with breast cancer.

The present application relates to an advantageous combination of a human anti-IGF antibody with exemestane and everolimus in patients with breast cancer.

## Brief Description of the Figures

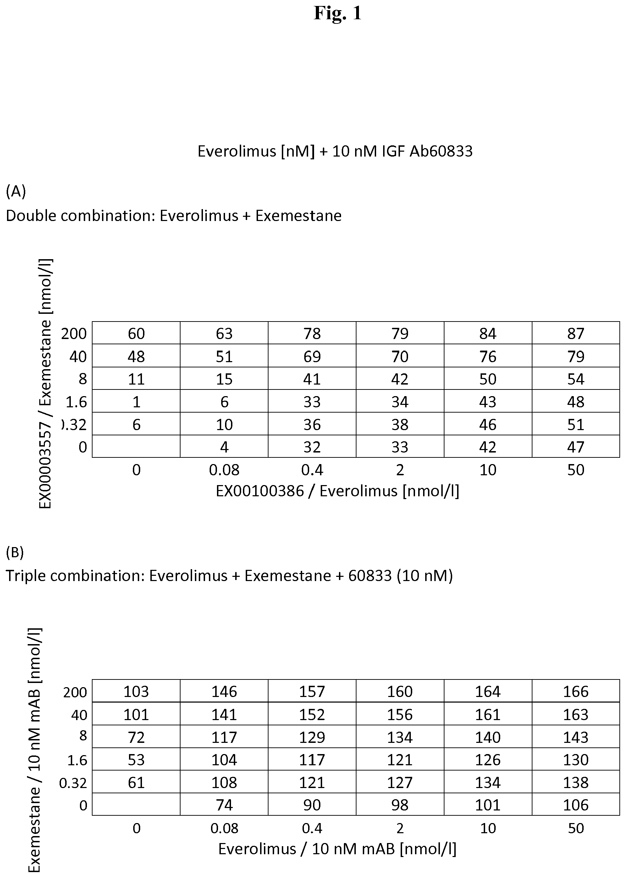

**Figure 1. Effect of a triple combination of IGF Ab 60833, everolimus and exemestane, against a double combination of everolimus and exemestane.** The figure shows a comparison of the double combination of everolimus and exemestane (top panel) against the triple combination of IGF Ab 60833, everolimus and exemestane (bottom panel). Fig. 1 A.: Double combination - Everolimus + Exemestane "Campaign "3x combi separated by mAB

concentration", N=3 avg(Bliss $CGI_m$) of AlamarBlue_cell line (144h,cell{29164/5000/}) Fig. 1 B.: Triple combination: Everolimus + Exemestane, + 60833 (10 nM)"Campaign "3x combi separated by mAB concentration", N=3 avg(Bliss $CGI_m$) of AlamarBlue_cell line (144h,cell{29164/5000/})

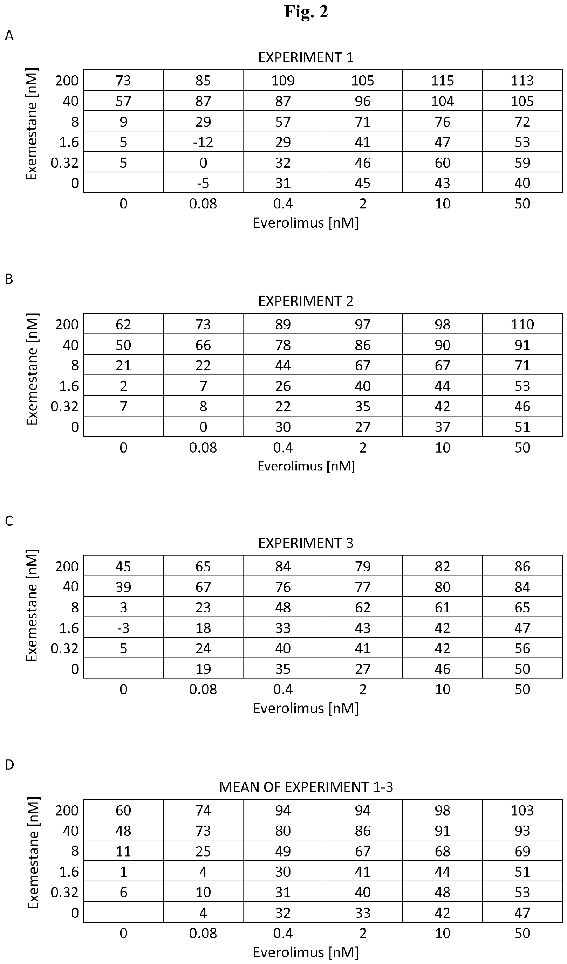

**Figure 2. Effect of everolimus and exemestane, alone or in combination, on the *in vitro* growth of MCF7aro cells.** In three independent experiments (A, B, C) MCF7aro cells were treated with different concentrations of everolimus and exemestane, as single agents and in combination. After incubation for 6 days the inhibitory effect on MCF7aro cell growth was quantified using the AlamarBlue® cell viability assay. The data shown in (D) represent the mean values of the three independent experiments.

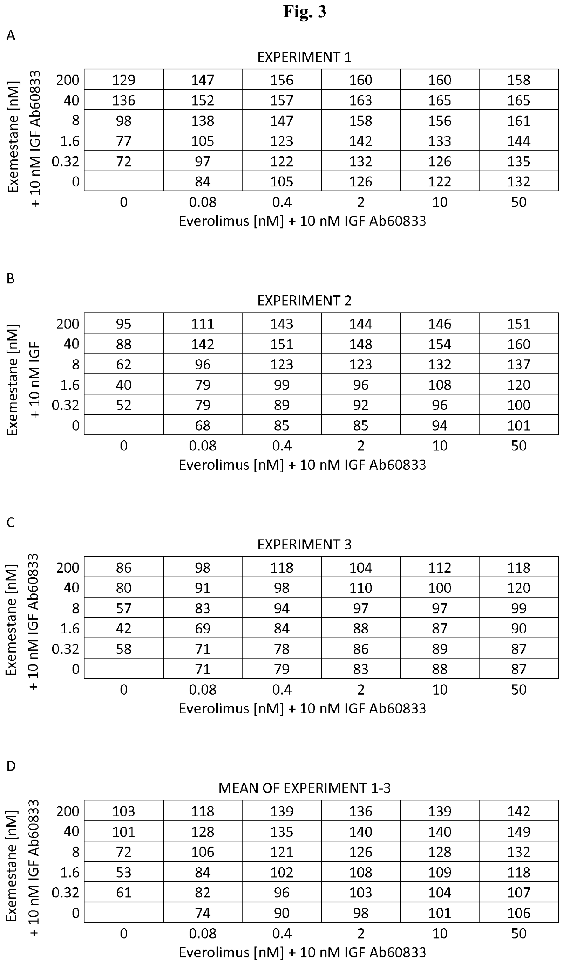

**Figure 3. Effect of everolimus and exemestane, alone or in combination, combined with 10 nM of IGF Ab 60833, on the *in vitro* growth of MCF7aro cells.** In three independent experiments (A, B, C) MCF7aro cells were treated with different concentrations of everolimus and exemestane, as single agents and in combination, combined with 10 nM of IGF Ab 60833. After incubation for 6 days the inhibitory effect on MCF7aro cell growth was quantified using the AlamarBlue® cell viability assay. The data shown in (D) represent the mean values of the three independent experiments

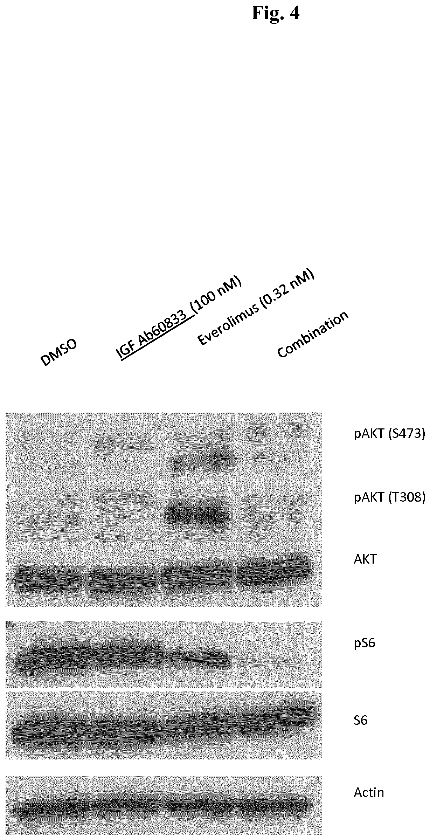

**Figure 4. Effect of IGF Ab 60833and everolimus, alone or in combination, on the AKT and S6 phosphorylation status in MCF7aro cells *in vitro.*** MCF7aro cells were treated with 100 nM **IGF Ab 60833** and 0.32 nM everolimus as single agents or in combination for 24 hours. Cell lysates prepared from vehicle (DMSO)-treated and inhibitor-treated cells were analysed by Western blotting

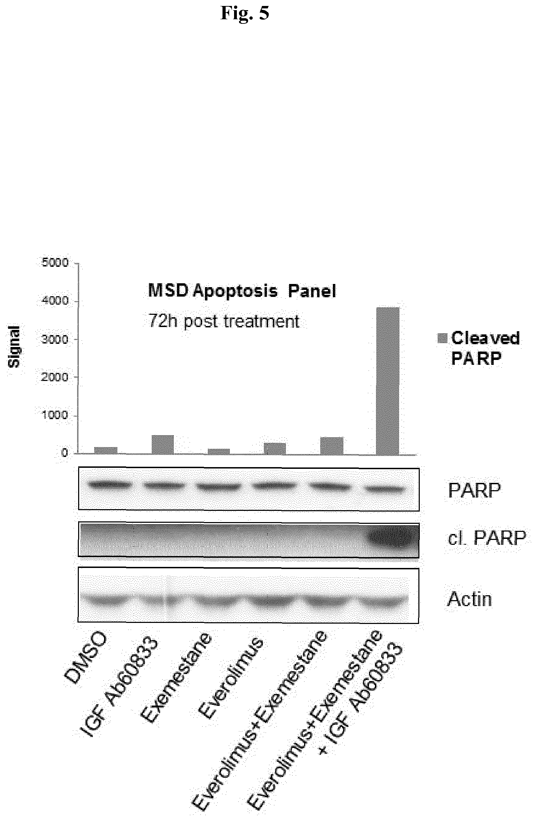

**Figure 5. Effect of IGF Ab 60833, exemestane and everolimus, alone or in combination, on induction of apoptosis in MCF7aro cells *in vitro.*** MCF7aro cells were treated with 1 μM **IGF Ab 60833,** 1 μM exemestane and 1 μM everolimus as single agents or in combination for 72 hours. Cell lysates prepared from vehicle (DMSO)-treated and inhibitor-treated cells were analysed by Western blotting and MSD Apoptosis Panel for induction of PARP cleavage

**Brief description of the invention**

In one aspect, the present invention pertains to an insulin-like growth factor (IGF) receptor antagonist for use in the treatment of patients with breast cancer in combination with exemestane and everolimus, which IGF receptor antagonist is an antibody having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy chain variable region of SEQ ID NO: 43 and a light chain variable region of SEQ ID NO: 44, or an antibody having a heavy chain of SEQ ID NO: 19, and a light chain of SEQ ID NO: 20, or an antibody having a heavy chain of SEQ ID NO: 29, and a light chain of SEQ ID NO: 30, or an antibody having a heavy chain of SEQ ID NO: 39, and a light chain of SEQ ID NO: 40.

In another aspect, described herein is a method of treatment of breast cancer comprising administering a therapeutically effective amount of an IGF receptor antagonist to a patient in need thereof, and additionally administering a therapeutically effective amount of an exemestane and everolimus to the same patient.

Preferably the breast cancer is locally advanced or metastatic breast cancer.

**Detailed description of the invention**

The present invention relates to the combination of an insulin-like growth factor (IGF) receptor antagonist with exemestane (Aromasin®) and the rapalog (first generation mTOR inhibitor) everolimus (Afinitor®) in breast cancer, specifically advanced estrogen receptor positive breast cancer. Mammalian target of rapamycin (mTOR) inhibitors mediate AKT activation through a type 1 insulin-like growth factor receptor (IGF-1R)-dependent mechanism. The combination

with insulin-like growth factor (IGF) receptor antagonist is thought to enhance mTOR-targeted anticancer activity by modulating resistance to mTOR inhibition by targeting feedback loops.

In one aspect, described herein is an insulin-like growth factor (IGF) receptor antagonist for use in the treatment of patients with breast cancer in combination with exemestane and everolimus.

Preferably the breast cancer is locally advanced.

Preferably the breast cancer is a metastatic breast cancer.

Methods of identifying whether a patient has a breast cancer is locally advanced or metastatic are well known in the art and can be readily used by the skilled person. In another embodiment, the locally advanced or metastatic breast cancer overexpresses hormone receptors, such as estrogen receptors.

In another embodiment, the locally advanced or metastatic breast cancer is positive for estrogen receptor (ER) and / or progesterone receptor (PgR). Preferably the locally advanced or metastatic breast cancer is also negative for HER2. Also preferably the locally advanced or metastatic breast cancer is also refactory to non-steroidal aromatase inhibitor (e.g. letrozole and/or anastrozole).

Methods of identifying whether a patient has a breast cancer that is positive for estrogen receptor (ER) and / or progesterone receptor (PgR), and whether it overexpresses hormone receptors, such as estrogen receptor, are well known in the art.

In another embodiment, the patient to be treated has locally advanced or metastatic breast cancer not deemed amenable to curative surgery or curative radiation therapy.

In another embodiment, the patient to be treated is a postmenopausal woman.

In another embodiment, the patient to be treated shows objective evidence of recurrence or progressive disease on or after the last line of systemic therapy for breast cancer prior to study entry.

In another embodiment, the patient to be treated has a measurable lesion according to RECIST version 1.1 or Bone lesions only: lytic or mixed (lytic + sclerotic) in the absence of measurable lesion as defined above.

In another embodiment, the patient to be treated has a Eastern Cooperative Oncology Group performance score <= 2.

In another embodiment, the patient to be treated has a life expectancy of >= 6 months in the opinion of the investigator.

In another embodiment, the patient to be treated has fasting plasma glucose < 8.9 mmol/L (< 160 mg/dL) and HbA1c < 8.0%.

In another embodiment the patient to be treated has not been treated with agents targeting on IGF pathway, phosphoinositide 3-kinase (PI3K) signaling pathway, protein kinase B (AKT), or mammalian target of rapamycin (mTOR) pathways.

In another embodiment the patient to be treated has not been treated with exemestane.

In another embodiment the patient to be treated does not have known hypersensitivity to monoclonal antibody, mTOR inhibitors (e.g. sirolimus), or to the excipients of any study drugs.

In another embodiment the patient to be treated does not have ovarian suppression by ovarian radiation or treatment with a luteinizing hormone-releasing hormone (LH-RH) agonist.

In another embodiment the patient to be treated has not less than one week after receiving immunization with attenuated live vaccines prior to treatment.

In another embodiment the patient to be treated has not received radiotherapy within 4 weeks prior to run-in treatment, except in case of localized radiotherapy for analgesic purpose or for lytic lesions at risk of fracture which can then be completed within two weeks prior to study treatment

In another embodiment the patient to be treated has not received chemotherapy, biological therapy (other than bevacizumab), immunotherapy or investigational agents within 5 half-life of the drug or within two weeks prior to the start of treatment, whichever is longer; bevacizumab treatment within 4 weeks prior to start of study treatment.

In another embodiment the patient to be treated has not received hormonal treatment for breast cancer within 2 weeks prior to start of treatment.

In another embodiment the patient to be treated has not received major surgery within 4 weeks before starting treatment or scheduled for surgery during the projected course of the treatment.

In another embodiment the patient to be treated is not receiving concomitant immunosuppressive agents or chronic corticosteroids use except Topical applications, inhaled sprays, eye drops or local injections or on stable low dose of corticosteroids for at least two weeks before study treatment.

In another embodiment the patient to be treated does not have chronic hepatitis B infection, chronic hepatitis C infection and/or is a known HIV carrier.

In another embodiment the patient to be treated does not show QTcF prolongation > 470 ms or QT prolongation deemed clinically relevant.

In another embodiment the patient to be treated does not show disease that is rapidly progressing or life threatening such as extensive symptomatic visceral disease including hepatic involvement and pulmonary lymphangitic

spread of tumor.

In another embodiment the patient to be treated does not have history of brain or other CNS metastases.

In another embodiment the patient to be treated does not have bilateral diffuse lymphangitic carcinomatosis.

In another embodiment the patient to be treated does not have hypokalemia of Grade >1.

In another embodiment the patient to be treated does not have history of another primary malignancy within 5 years, with the exception of adequately treated in-situ carcinoma of the cervix, uteri, basal or squamous cell carcinoma or non-melanomatous skin cancer.

In another embodiment the patient to be treated does not have family history of long QT syndrome.

In another embodiment the patient to be treated does not have any concomitant serious illness or organ system dysfunction which would either compromise patient safety or interfere with the of the safety and anti-tumor activity of the medicaments.

In another embodiment the patient to be treated is not being treated with drugs recognized being strong or moderate CYP3A4 and/or PgP inhibitors and/or strong CYP3A4 inducers within 2 weeks prior to treatment.

In another embodiment the patient to be treated has not received more than two lines of chemotherapy for locally advanced or metastatic breast cancer.

An IGF receptor antagonist within the context of the invention is a compound that interferes with, either directly or indirectly, and reduces or blocks IGF receptor signaling. Preferaby, an IGF receptor antagonist is a compound that reduces or blocks binding of IGF ligand to its receptor, or inhibits the tyrosine kinase activity of the IGF receptor.

In a further embodiment, the IGF receptor antagonist described herein is an antibody that binds to IGF ligand and thus reduces or prevents binding of the ligand to the receptor. In another embodiment, the IGF receptor antagonist is an antibody that binds to the IGF-1 receptor and thus reduces or prevents binding of the ligand to the receptor. By blocking receptor-ligand binding, ligand-induced receptor signaling through the tyrosine kinase activity of the receptor is reduced or prevented. Such antibodies are generally referred to as neutralizing antibodies. In another aspect, the IGF receptor antagonist described herein neutralizes the growth promoting properties of the insulin-like growth factors, IGF-1 and IGF-2.

The term "antibody" encompasses antibodies, antibody fragments, antibody-like molecules and conjugates with any of the above. Antibodies include, but are not limited to, poly- or monoclonal, chimeric, humanized, human, mono-, bi- or multispecific antibodies. The term "antibody" shall encompass complete immunoglobulins as they are produced by lymphocytes and for example present in blood sera, monoclonal antibodies secreted by hybridoma cell lines, polypeptides produced by recombinant expression in host cells, which have the binding specificity of immunoglobulins or monoclonal antibodies, and molecules which have been derived from such immunoglobulins, monoclonal antibodies, or polypeptides by further processing while retaining their binding specificity. In particular, the term "antibody " includes complete immunoglobulins comprising two heavy chains and two light chains. In another embodiment, the term encompasses a fragment of an immunoglobulin, like Fab fragments. In another embodiment, the term "antibody" encompasses a polypeptide having one or more variable domains derived from an immunobulin, like single chain antibodies (scFv), single domain antibodies, and the like.

In a further embodiment, the IGF receptor antagonist described herein is an antibody against IGF-1, an antibody against IGF-2, an antibody binding both IGF-1 and IGF-2, an antibody against IGF-1 receptor (IGF-1R), or an inhibitor of IGF-1R tyrosine kinase activity.

In another embodiment, the IGF receptor antagonist is an IGF ligand antibody having heavy chain complementary determining regions of SEQ ID NO: 1 (HCDR1), SEQ ID NO: 2 (HCDR2), and SEQ ID NO: 3 (HCDR3) and light chain determining regions of SEQ ID NO: 4 (LCDR1), SEQ ID NO: 5 (LCDR2), and SEQ ID NO: 6 (LCDR3).

In another embodiment, the IGF receptor antagonist is an IGF ligand antibody having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3).

In another embodiment, the IGF receptor antagonist is an IGF ligand antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3).

In another embodiment, the IGF receptor antagonist is an IGF ligand antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3).

In another embodiment, the IGF receptor antagonist is an IGF ligand antibody having a heavy chain variable region of SEQ ID NO: 7 and a light chain variable region of SEQ ID NO: 8.

In another embodiment, the IGF receptor antagonist is an IGF ligand antibody having a heavy chain variable region of SEQ ID NO: 17 and a light chain variable region of SEQ ID NO: 18.

In another embodiment, the IGF receptor antagonist is an IGF ligand antibody having a heavy chain variable region of SEQ ID NO: 27 and a light chain variable region of SEQ ID NO: 28.

In another embodiment, the IGF receptor antagonist is an IGF ligand antibody having a heavy chain variable

region of SEQ ID NO: 37 and a light chain variable region of SEQ ID NO: 38.

In another embodiment, the IGF receptor antagonist is an IGF ligand antibody having a heavy chain variable region of SEQ ID NO: 41 and a light chain variable region of SEQ ID NO: 42.

In another embodiment, the IGF receptor antagonist is an IGF ligand antibody having a heavy chain variable region of SEQ ID NO: 43 and a light chain variable region of SEQ ID NO: 44.

In another embodiment, the IGF receptor antagonist is an IGF ligand antibody having a heavy chain of SEQ ID NO: 9, and a light chain of SEQ ID NO: 10.

In another embodiment, the IGF receptor antagonist is an IGF ligand antibody having a heavy chain of SEQ ID NO: 19, and a light chain of SEQ ID NO: 20.

In another embodiment, the IGF receptor antagonist is an IGF ligand antibody having a heavy chain of SEQ ID NO: 29, and a light chain of SEQ ID NO: 30.

In another embodiment, the IGF receptor antagonist is an IGF ligand antibody having a heavy chain of SEQ ID NO: 39, and a light chain of SEQ ID NO: 40.

In another embodiment, the IGF receptor antagonist is an IGF receptor antibody having a heavy chain of SEQ ID NO: 45, and a light chain of SEQ ID NO: 46.

Preferably the the IGF receptor antagonist is 60833, an antibody against IGF ligand having a heavy chain of SEQ ID NO: 39 and a light chain of SEQ ID NO: 40. Its manufacture has been disclosed in WO 2010/066868.

In another embodiment, the IGF receptor antagonist is dusigitumab, figitumumab, dalotuzumab, cixutumumab, robatumumab, or ganitumab.

In another embodiment, the IGF receptor antagonist is linsitinib.

Manufacture and therapeutic use of the aforementioned antibodies is disclosed in the art and are well known to the skilled person, and specific disclosures can be identified in WO2002/53596, WO2007/070432, WO2008/152422, WO2008/155387, and WO2010/066868.

In one embodiment, the antibody is produced by recombinant expression in a mammalian host cell, purified by a series of chromatographic and non-chromatographic steps, and formulated in an aqueous buffer composition for parenteral (intravenous) infusion or injection at an antibody concentration of 10 mg/ml, said buffer comprising for example 25 mM Na citrate pH 6, 115 mM NaCl, and 0.02 % polysorbate 20. For intravenous infusion, the pharmaceutical composition may be diluted with a physiological solution, e.g. with 0.9 % sodium chloride or G5 solution.

The antibody may be administered to the patient at a dose between 1 mg/kg to 20 mg/kg, by one or more separate administrations, or by continuous infusion, e.g. infusion over 1 hour. A typical treatment schedule usually involves administration of the antibody once every week to once every three weeks. For example, a weekly dose could be 5, 10, or 15 mg/kg.

The antibody may also be administered to the patient at a dose of between 500 and 1000mg per week, optionally 750 or 1000mg per week.

The IGF receptor antagonist is administered to the patient in combination with administration of exemestane and everolimus. "In combination" means that the drugs are administered to the same patient within a certain time frame to achieve a therapeutic effect caused by the combined effects of modes of action. In one aspect, exemestane and everolimus are administered on the same day as the IGF receptor antagonist. In another aspect, exemestane and everolimus are administered one, two, three, four, five, six or seven days before or after admistration of the IGF receptor antagonist.

In another embodiment, all three active compounds are present within the same pharmaceutical composition. Hence, in another embodiment, described herein is a pharmaceutical composition, comprising an IGF receptor antagonist and exemestane and everolimus, together with a pharmaceutically acceptable carrier.

Exemestane is a member of the class of drugs known as aromatase inhibitors. Some breast cancers require estrogen to grow. Those cancers have estrogen receptors (ERs), and are called ER-positive. They may also be called estrogen-responsive, hormonally-responsive, or hormone-receptor-positive. Aromatase is an enzyme that synthesizes estrogen. Aromatase inhibitors block the synthesis of estrogen. This lowers the estrogen level, and slows the growth of cancers.

The structure of exemestane is provided below

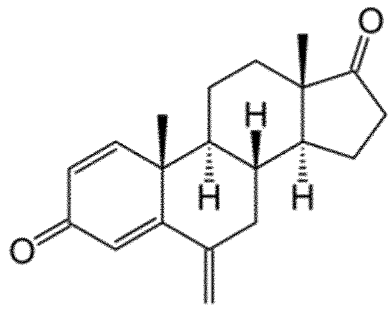

The systematic name is 6-Methylideneandrosta-1,4-diene-3,17-dione

Exemestane can be obtained commerically under the trade name Aromasin. Manufacture, formulation, and use of the exemestane can be found in the state of the art.

Preferably exemestane will be supplied to patients orally at a dosage of 25mg per day;

Everolimus is an inhibitor of mammalian target of rapamycin (mTOR).

The structure of everolimus is provided below

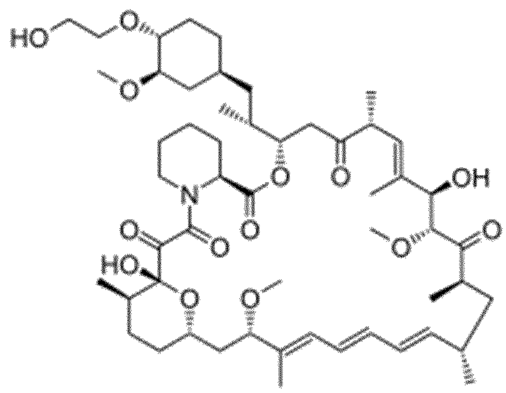

The systematic name is dihydroxy-12-[(2R)-1-[(1S,3R,4R)-4-(2-hydroxyethoxy)-3-methoxycyclohexyl]propan-2-yl]-19,30-dimethoxy-15,17,21,23,29,35-hexamethyl-11,36-dioxa-4-azatricyclo[30.3.1.0 hexatriaconta-16,24,26,28-tetraene-2,3,10,14,20-pentone

Everolimus can be obtained commerically under the trade name Afinitor. Manufacture, formulation, and use of the exemestane can be found in the state of the art.

Preferably everolimus will be supplied to patients orally at a dosage of between 5mg and 10mg per day; optionally 7.5 mg per day;

In another embodiment, described herein is a method of treatment of breast cancer comprising administering a therapeutically effective amount of an IGF receptor antagonist to a patient in need thereof, and additionally administering a therapeutically effective amount of an exemestane and everolimus to the same patient.

Preferably the breast cancer is locally advanced or metastatic breast cancer.

A "therapeutically effective amount" of the IGF receptor antagonist or exemestane and everolimus to be administered is the minimum amount necessary to prevent, ameliorate, or treat a locally advanced or metastatic breast cancer.

**Example 1: Study of IGF Ab 60833 in Combination with Exemestane and Everolimus Versus Exemestane and Everolimus Alone in Women with Locally Advanced or Metastatic Breast Cancer.**

*Introduction*

The study proposed here investigates the effect of IGF Ab 60833 in combination with Exemestane and Everolimus in in estrogen receptor positive metastatic breast cancer.

In more detail, the phase I part will determine the Maximum Tolerated Dose (MTD) and Recommended Phase II Dose (RP2D) of IGF Ab 60833 and everolimus incombination with exemestane in women with HR+/HER2- advanced breast cancer. The Phase II part will evaluate the antitumor activity of IGF Ab 60833 in combination with exemestane and everolimus compared to exemestane and everolimus alone in women with HR+/HER2- advanced breast cancer.

*Background*

IGF Ab 60833 is a fully human monoclonal antibody (HumAb) of the IgG1 isotype. The Ab binds with high affinity to IGF-1 and IGF-2, and potently neutralizes the proliferative and prosurvival cellular signaling triggered by both proteins.

Everolimus is a selective mTOR (mammalian target of rapamycin) inhibitor.

Exemestane is an oral steroidal aromatase inhibitor that is used in ER-positive breast cancer in addition to surgery and/or radiation in post-menopausal women.

*Administration*

IGF Ab 60833 will be supplied to the study sites as a concentrate for solution for injection/infusion. A total of 1000 mg milligram(s) will be supplied to patients intravenously. The patient will have continuous treatment until disease progression, intolerable AEs, consent withdrawalor non-compliance with the study.

Everolimus will be supplied to patients orally at a dosage of 10 mg milligram per day. The patient will have continuous treatment until disease progression, intolerable AEs, consent withdrawalor non-compliance with the study.

Exemestane will also be supplied to patients orally.

*Medical condition or disease under investigation*

The study proposed here will investigate Locally Advanced or Metastatic Breast Cancer positive for estrogen-receptor(ER) and/or progesterone receptor (PgR) and negative for HER2 which is refractory to non-steroidal aromatase inhibitor (letrozole and/or anastrozole).

*Principle inclusion criteria*

The following criteria will be used to evaluate the inclusion of patients in to the study.

- Histologically-confirmed locally advanced (aBC) or metastatic breast cancer (mBC) not deemed amenable to curative surgery or curative radiation therapy
- Tumors are positive for estrogen-receptor (ER) and/or progesterone receptor (PgR).
- Tumors must be negative for HER2 per local lab testing.
- Must have adequate archival tumor tissue from surgery or biopsy.
- Postmenopausal women.
- Objective evidence of recurrence or progressive disease on or after the last line of systemic therapy for breast cancer prior to study entry
- The patient is disease refractory to non-steroidal aromatase inhibitor (letrozole and/or anastrozole)
- Patients must have Measurable lesion according to RECIST version 1.1 or Bone lesions only: lytic or mixed (lytic + sclerotic) in the absence of measurable lesion as defined above
- Eastern Cooperative Oncology Group performance score $<= 2$.
- Life expectancy of $>= 6$ months in the opinion of the investigator
- Fasting plasma glucose < 8.9 mmol/L (< 160 mg/dL) and HbA1c < 8.0%
- Adequate organ function
- Recovered from any previous therapy related toxicity to <= Grade 1 at study entry (except for stable sensory neuropathy <=Grade 2 and alopecia)
- Written informed consent that is consistent with ICH-GCP guidelines and local regulations Inclusion criteria for the biopsy substudy are identical to the main study of the phase II part except for the following two inclusion criteria:
- Fresh tumor biopsy should be taken when deemed safe and feasible by the investigator and upon informed consent by the patient. Bone lesion is not recommended for biopsy
- Patients eligible to undergo tumor biopsy should have normal coagulation parameters (INR and PTT within normal range)

*Principle exclusion criteria*

The following criteria will be used to evaluate the exclusion of patients in to the study.

- Previous treatment with agents targeting on IGF pathway, phosphoinositide 3-kinase (PI3K) signaling pathway, protein kinase B (AKT), or mammalian target of rapamycin (mTOR) pathways

- Prior treatment with exemestane
- Known hypersensitivity to monoclonal antibody, mTOR inhibitors (e.g. sirolimus), or to the excipients of any study drugs
- Ovarian suppression by ovarian radiation or treatment with a luteinizing hormone-releasing hormone (LH-RH) agonist
- Less than one week after receiving immunization with attenuated live vaccines prior to study treatment
- Radiotherapy within 4 weeks prior to run-in treatment, except in case of localized radiotherapy for analgesic purpose or for lytic lesions at risk of fracture which can then be completed within two weeks prior to study treatment
- Chemotherapy, biological therapy (other than bevacizumab), immunotherapy or investigational agents within 5 half-life of the drug or within two weeks prior to the start of study treatment, whichever is longer; bevacizumab treatment within 4 weeks prior to start of study treatment
- Hormonal treatment for breast cancer within 2 weeks prior to start of study treatment
- Major surgery in the judgement of the investigator within 4 weeks before starting study treatment or scheduled for surgery during the projected course of the study
- Patients receiving concomitant immunosuppressive agents or chronic corticosteroids use except Topical applications, inhaled sprays, eye drops or local injections or Patients on stable low dose of corticosteroids for at least two weeks before study entry
- Chronic hepatitis B infection, chronic hepatitis C infection and/or known HIV carrier
- QTcF prolongation > 470 ms or QT prolongation deemed clinically relevant by the investigator
- Disease that is considered by the investigator to be rapidly progressing or life threatening such as extensive symptomatic visceral disease including hepatic involvement and pulmonary lymphangitic spread of tumor
- History of brain or other CNS metastases
- Bilateral diffuse lymphangitic carcinomatosis
- Hypokalemia of Grade >1
- History of another primary malignancy within 5 years, with the exception of adequately treated in-situ carcinoma of the cervix, uteri, basal or squamous cell carcinoma or non-melanomatous skin cancer
- Family history of long QT syndrome
- Any concomitant serious illness or organ system dysfunction which in the opinion of the investigator would either compromise patient safety or interfere with the evaluation of the safety and anti-tumor activity of the test drug(s)
- Patients being treated with drugs recognized being strong or moderate CYP3A4 and/or PgP inhibitors and/or strong CYP3A4 inducers within 2 weeks prior to study entry
- Patients received more than two lines of chemotherapy for locally advanced or metastatic breast cancer (For the Phase II: more than one line)

*End points*

The primary end points are:

1: Progression-free survival (PFS), the timepoint of evaluation being up to 10,8 months.

2: occurrence of Dose Limiting Toxicity (DLT) - phase I part, the timepoint of evaluation being up to 28 days.

The secondary End Points are:

1: Time to progression (TTP), defined as the duration of time from the date of C1V1 until the date of the first objective tumor progression, the timepoint of evaluation being up to 10,8 months.

2: Objective response (OR), defined as complete response (CR) or partial response (PR) (CR + PR), the timepoint of evaluation being up to 10,8 months.

3: Time to objective response, the timepoint of evaluation being up to 10,8 months.

4: Duration of objective response, the timepoint of evaluation being up to 10,8 months.

5: Clinical benefit (CB), defined as best overall response of complete response (CR) or partial response (PR), or stable disease (SD) >=6 months, or Non-CR/Non-PD for >=6 months (CR + PR + SD6m + Non-CR/Non-PD6m), the timepoint of evaluation being up to 10,8 months.

6: Duration of clinical benefit, the timepoint of evaluation being up to 10,8 months.

**Example 2: Preliminary study of IGF Ab 60833 in Combination with Exemestane and Everolimus Versus Exemestane and Everolimus Alone in Women with Locally Advanced or Metastatic Breast Cancer.**

Preliminary investigations were performed in to the effect of a triple combination of IGF Ab 60833, everolimus and exemestane, against a double combination of everolimus and exemestane. The investigation was conducted in ER positive breast cancer cell line engineered to expression the human aromatase gene.

Figure 1 shows a comparison of the double combination of everolimus and exemestane (top panel) against the triple combination of IGF Ab 60833, everolimus and exemestane (bottom panel).

It can be clearly seen from a comparison of the two panels that IGF Ab 60833 causes a surprisingly large increase in cell growth inhibition to the everolimus and exemestane combination.

**Example 3: Effect of IGF Ab 60833 in combination with the mTOR inhibitor everolimus and the aromatase inhibitor exemestane on growth and biomarker phosphorylation status of the breast cancer cell line MCF7aro**

**SUMMARY**

The aim of the present study was to explore the *in vitro* effect of the combination of IGF Ab 60833, a fully human antibody that binds to IGF-1 and IGF-2, with the mTORC1 inhibitor everolimus and the aromatase inhibitor exemestane on the proliferation of MCF7aro cells, derived from the estrogen receptor positive breast cancer cell line MCF7, engineered to stably express the human aromatase protein.

MCF7aro breast cancer cells were cultured in steroid-deprived medium, supplemented with the estradiol precursor androstenedione and incubated with IGF Ab 60833, everolimus and exemestane as single agents or in combination, to determine effects on PI3K/mTOR pathway signaling, cell proliferation and survival.

Whereas treatment of MCF7aro cells with everolimus alone resulted in enhanced AKT phosphorylation, co-treatment with IGF Ab 60833 prevented the increase in phospho AKT and led to more pronounced inhibition of downstream signaling. The triple combination of IGF Ab 60833 with everolimus and exemestane resulted in enhanced cell growth inhibition and induction of apoptosis as compared to treatment with the dual combination of everolimus and exemestane.

This study has demonstrated that addition of IGF Ab 60833 to the combination of everolimus and exemestane, which is the current standard-of-care treatment in hormone receptor positive breast cancer, leads to improved anti-neoplastic activity *in vitro.*

**INTRODUCTION**

Both the insulin-like growth factor (IGF) signalling system as well as the PI3 kinase/mTOR signaling pathway plays an important role in the regulation of survival and proliferation of mammalian cells.

The insulin-like growth factor (IGF) signalling system consists of ligands (insulin-like growth factors 1 and 2 (IGF-1, IGF-2), IGF-binding proteins (IGFBPs), and receptors (insulin-like growth factor 1 receptor (IGF-1R), IGF-2R, and insulin receptor (IR). Evidence that targeting IGF may be useful in cancer treatment was first recognised decades ago. Research in IGF signalling has shown that it controls key cellular activities, including proliferation, growth, and survival and is often deregulated in neoplasia.

Expression of IGF-IR has been shown to be increased in a variety of cancers, including lung, colon, prostate, breast, ovarian, liver cancer, and sarcomas. The IGF system has thus become a target for anti-cancer drug development. Pharmacologic targeting strategies include inhibition of receptor function with IGF-1R antibodies or small molecule receptor tyrosine kinase inhibitors. IGF Ab 60833 offers an alternative approach by acting as an IGF ligand neutralizing antibody. In addition to expression of the IGF-1R, there is evidence for autocrine and/or paracrine expression of the IGF ligands, particularly autocrine IGF-2, in multiple cancers. IGF-2 has also been shown to bind and signal through the insulin receptor isoform A (IR-A) which is expressed on foetal and cancer cells. Thus, the strategy of targeting IGF-1 and IGF-2 has the potential for therapeutic advance.

IGF Ab 60833 is a fully human monoclonal antibody which binds IGF-1 and IGF-2 and neutralizes their biological effects by blocking the interaction with their cognate receptors, with the potential of anti-neoplastic activity.

A large number of pre-clinical studies have evaluated the functional role of the serine/threonine kinase mTOR and other components of the PI3 kinase/mTOR pathway in cancer. Activation of the PI3 kinase/mTOR signalling pathway by mutation or amplification of pathway components has been found in a large proportion of cancers of different origin, suggesting an important role of pathway hyperactivation in the aetiology of the disease.

The mTOR kinase functions in two cellular multi-protein complexes, mTORC1 and mTORC2, with distinct substrates and mechanisms of activation, and regulates survival, growth and cell cycle progression of cells.

Hyperactivation of the PI3K/mTOR pathway has been claimed to result in resistance to standard therapy, e.g. to endocrine therapy in hormone receptor positive breast cancer.

Inhibition of mTOR, in combination with other agents, is therefore considered to be an attractive approach to cancer therapy.

Rapamycin derivatives (rapalogs) have been approved for the treatment of several cancer types. They act as allosteric inhibitors of the mTORC1 protein complex. The rapalog everolimus (Afinitor®, Novartis Pharma), in combination with endocrine therapy, i.e. the aromatase inhibitor exemestane (Aromasin®, Pfizer Inc.), has been approved for the treatment of estrogen receptor (ER)-positive breast cancer.

Despite the fact that rapamycin analogues such as everolimus have shown clinical activity in several cancers, pre-clinical and clinical data suggest that, when mTORC1 is blocked, there may be acquired resistance by release of a negative feedback loop, resulting in induction of AKT phosphorylation, i.e. re-activation of PI3 kinase/mTOR pathway signaling.

In a preclinical model of Ewing's sarcoma, improved anti-tumor efficacy was observed when rapamycin was combined with IGF Ab 60833. IGF Ab 60833 was shown to inhibit the rapamycin induced increase in pAKT indicating that the elevated pAKT level was due to enhanced IGF ligand driven signalling.

Together, preclinical data as well as clinical evidence indicate that combination of agents targeting more than one pathway component, known as vertical pathway inhibition, results in a more pronounced blockade and improved anti-cancer efficacy.

## OBJECTIVES

The aim of the present study was to explore the *in vitro* effect of the combination of IGF Ab 60833, with the mTORC1 inhibitor everolimus and the aromatase inhibitor exemestane on the proliferation of MCF7aro cells, derived from the estrogen receptor positive breast cancer cell line MCF7, engineered to stably express the human aromatase protein.

## STUDY DESIGN

Three independent experiments were conducted to determine the anti-proliferative effect of IGF Ab 60833 in combination with everolimus and exemestane in MCF7aro breast cancer cells. Cell proliferation and viability following 6 days of incubation with test compounds was determined using an assay that monitors the reducing environment of the living cells in each assay well.

The effect on PI3 kinase/mTOR pathway signalling was assessed by Western Blot analysis, induction of apoptosis was monitored using the Meso Scale Discovery Apoptosis Whole Cell Lysate Kit.

IGF Ab 60833 comprises a heavy chain of SEQ ID NO: 39 and a light chain of SEQ ID NO: 40. Its manufacture has been disclosed in WO 2010/066868.

Everolimus (EX00100386) as used in this study has the chemical structure provided in herein.

Exemestane (EX0003557) as used in this study has the chemical structure provided in herein.

MCF7aro cells stably express the human aromatase protein. They were derived from MCF7 human ER-positive breast adenocarcinoma cells (ATCC, HTB-22), by transfection of aromatase cDNA, Geneticin selection (neomycin) and clonal purification.

## METHODS

*Cell culture*

MCF7aro cells were cultivated in MEM growth medium supplemented with 10% FBS, 2 mM GlutaMAX, 1 mM sodium pyruvate and 0.1 mg/ml Geneticin as monolayer cultures. The cells were maintained in 175 $cm^2$ tissue culture flasks at 37°C and 5% $CO_2$ in a humidified atmosphere.

For cell proliferation assays and Western Blot analysis the cells were steroid-deprived for 72 h by cultivation in starvation medium (MEM alpha without phenol red, supplemented with 10% charcoal stripped FBS, 2 mM GlutaMAX and 1 mM sodium pyruvate).

*Cell proliferation assay of adherently growing MCF7aro breast cancer cells*

This assay was used to determine the inhibitory effect of IGF Ab 60833, everolimus and exemestane on the viability and growth of MCF7aro cells.

Assay conditions: Adherent cells were detached with Trypsin/EDTA solution, resuspended in starvation medium and diluted to 25,000 cells per ml in starvation medium. 200 $\mu$l cell suspension (5,000 cells) per well were plated in four sterile Nunc™ Edge 96-well plates (except wells B1/B2: medium control, add only 200 $\mu$l starvation medium). Plates

were incubated for 48 h in a humidified incubator at 37°C and 5% $CO_2$. Two days later supernatants were aspirated and 150 μl assay medium (MEM alpha, without phenol red, supplemented with 10% charcoal stripped FBS, 2 mM GlutaMAX, 1 mM sodium pyruvate and 1 nM androstenedione) was added to each well in each plate.

Addition of test compounds: For the combination of everolimus with exemestane, 50 μl/well of assay medium was added to all wells. 5-fold serial dilutions of everolimus (highest test concentration 50 nM), exemestane (highest test concentration 200 nM) or DMSO (vehicle-treated cell control and medium control) were added to the cells using the HP D300 Digital Dispenser.

For the triple combination of everolimus, exemestane and IGF Ab 60833, 40 nM of IGF Ab 60833 solution was prepared in assay medium. 50 μl/well of IGF Ab 60833 solution was added to the cells to yield a final test concentration of 10 nM. 50 μl/well assay medium was added to cell control and medium control wells. 5-fold serial dilutions of everolimus (highest test concentration 50 nM) and exemestane (highest test concentration 200 nM), or DMSO were added to the cells using the HP D300 Digital Dispenser. The final volume per well was 200 μl.

The final concentration of the solvent DMSO in the test wells was 0.1%. Everolimus and exemestane were tested at 5 concentrations, each measured in duplicate wells, as single agents or in combination.

After 2 days of incubation with test compounds in a humidified incubator at 37°C and 5% $CO_2$, medium was changed to fresh assay medium and compounds were added again. After 6 days of total incubation time with test compounds, cells were stained with 20 μl of AlamarBlue® Cell Viability Reagent to assess cell viability. The plates were incubated for 6 hours at 37°C and 5% $CO_2$ to allow cells to convert resazurin to resorufin. Total fluorescence intensity of each well was then measured in a Wallac VICTOR Multilabel Counter using an excitation wavelength of 544 nm and measuring emission at 590 nm.

At the time of test compound addition, "time zero" (t=0) untreated cell plates were stained with AlamarBlue® Cell Viability Reagent. 50 μL of assay medium and 20 μl of AlamarBlue® Cell Viability Reagent were added to each well containing cells in 150 μl assay medium. After incubation for 6 hours at 37°C and 5% $CO_2$, total fluorescence intensity was measured.

The AlamarBlue® assay is designed to measure quantitatively the viability of cells by incorporating a fluorometric/colorimetric growth indicator based on the detection of metabolic activity. The fluorescent signal is proportional to total number of viable cells

When cells are alive they maintain a reducing environment within the cytosol. Resazurin, the active ingredient of the AlamarBlue® Cell Viability Reagent, is a nontoxic, cell permeable compound that is blue in colour and virtually non-fluorescent. Upon entering cells, resazurin is reduced to resorufin, which is red in colour and highly fluorescent. Viable cells continuously convert resazurin to resorufin, increasing the overall fluorescence and colour of the media surrounding cells.

Data analysis: The AlamarBlue® assay output for vehicle-treated control cells after 6 days of incubation, corresponding to 100% cell viability, was taken as the reference signal for all subsequent calculations. Relative cell viability in compound-treated cultures (signal percent of control, "POC") was calculated according to the following formula: POC (t=144 h) = 100 * fluorescence (compound wells) / fluorescence (control wells). In addition, for each compound-treated culture, the fluorescence signal after incubation for 144 hours (POC (t=144 h)) was related to the signal at the start of treatment (POC (t=0 h)): POC (t=0 h) = 100 * fluorescence at t = 0 (control wells) / fluorescence at t = 144 h (control wells).

To calculate concentration-response curves, the POC data were analyzed using a four-parameter log-logistic function without any upper or lower limitation. Relative cell growth inhibition (CGI %) in compound-treated cultures is calculated according to the following formula:

$$\%\ CGI^{144h} = \begin{cases} S_t^{144} \geq S_c^{0}: & \left[ 1 - \dfrac{S_t^{144h} - S_c^{0h}}{S_c^{144h} - S_c^{0h}} \right] \times 100\% \\ S_t^{144} < S_c^{0}: & \left[ 1 - \dfrac{S_t^{144h} - S_c^{0h}}{S_c^{0h}} \right] \times 100\% \end{cases}$$

$S_t^{144} = POC_{(t=144\ h)}$
$S_c^{0} = POC_{(t=0\ h)}$

A CGI of >0% and <100% reflects a partial growth-inhibitory effect relative to vehicle-treated controls, a CGI of 100% is equivalent of complete blockade of growth, and a CGI of >100% is indicative of net cell death.

Results are evaluated by a CGI matrix, plotting multiple compound concentrations of test compound 1 against different concentrations of test compound 2.

*Preparation of cell lysates and Western Blot (assessment of AKT and S6 phosphorylation status)*

1.8 x $10^6$ MCF7aro cells were plated in 10 cm dishes in starvation medium (MEM alpha without phenol red, supplemented with 10% charcoal stripped FBS, 2 mM GlutaMAX and mM sodium pyruvate). After overnight incubation, medium was changed to assay medium (MEM alpha without phenol red, supplemented with 10% charcoal stripped FBS, 2 mM GlutaMAX, 1 mM sodium pyruvate and 1 nM androstenedione) and the cells were treated with 100 nM of IGF Ab 60833 or 0.32 nM of everolimus or a combination of antibody and mTORC1 inhibitor. As a control, cells were treated with vehicle (DMSO) only. At 24 h post treatment, the cells were lysed on ice with MSD Tris Lysis Buffer containing 20 mM Tris pH 7.5, 150 mM NaCl, 1 mM EDTA, 1 mM EGTA, 1 % Triton X-100, completed with protease and phosphatase inhibitor cocktails. Prior to use, freshly prepared 2 mM PMSF was added to the buffer.

Total protein was isolated and protein concentration was quantified by Bradford protein assay according to the manufacturer's instructions.

30 μg of total protein was separated on a 4-12% Bis-Tris precast gel and blotted on a PVDF membrane with the BIO-RAD Trans-Blot® Turbo™ Instrument.

Membranes were blocked for 1 h in 5% skim milk in 1×TBS/0.1% Tween 20 at room temperature and then probed overnight at 4°C with antibodies against the following proteins: pAKT (S473), pAKT (T308), AKT, pS6 (S235/236), S6, and Actin, which served as loading control. Antibody dilutions were prepared in 5% skim milk. After washing and incubation with secondary antibody, the immunoblotted proteins were visualized using the ECL Western blotting detection reagent according to the manufacturer's instructions.

Primary antibodies:

rabbit anti phospho AKT (S473 (Cell Signaling #9271, 1:1000)
rabbit anti phospho AKT (T308) (Cell Signaling #2965, 1:1000)
rabbit anti phospho S6 Ribosomal Protein (S235/236) (Cell Signaling #2211, 1:2000)
rabbit anti AKT (Cell Signaling #9272, 1:2000)
rabbit anti S6 Ribosomal Protein (Cell Signaling #2217, 1:2000)
rabbit anti beta Actin (Dako #P0448, 1:1000)

Secondary antibody:
goat anti rabbit IgG , HRP conjugated (Dako #P0448, 1:1000)

*Preparation of cell lysates and Western blot (assessment of cleaved PARP)*

1.5 x $10^6$ MCF7aro cells were plated in 10 cm dishes in starvation medium (MEM alpha without phenol red, supplemented with 10% charcoal stripped FBS, 2 mM GlutaMAX and 1 mM sodium pyruvate). After overnight incubation, medium was changed to assay medium (MEM alpha without phenol red, supplemented with 10% charcoal stripped FBS, 2 mM GlutaMAX, 1 mM sodium pyruvate and 1 nM androstenedione) and the cells were treated with vehicle (DMSO), or 1 μM of IGF Ab 60833, or 1 μM exemestane, or 1 μM everolimus, or a combination of exemestane and everolimus, or a combination of all three inhibitors. At 72 h post treatment, the cells were lysed on ice with MSD Tris Lysis Buffer containing 20 mM Tris pH 7.5, 150 mM NaCl, 1 mM EDTA, 1 mM EGTA, 1 % Triton X-100, completed with protease and phosphatase inhibitor cocktails. Prior to use, freshly prepared 2 mM PMSF was added to the buffer.

Total protein was isolated and protein concentration was quantified by Bradford protein assay according to the manufacturer's instructions.

20 μg of total protein was separated on a 4-12% Bis-Tris precast gel and blotted on a PVDF membrane with the BIO-RAD Trans-Blot® Turbo™ Instrument.

Membranes were blocked for 1 h in 5% skim milk in 1×TBS/0.1% Tween 20 at room temperature and then probed overnight at 4°C with antibodies against PARP and Actin, which served as loading control. Antibody dilutions were prepared in 5% skim milk. After washing and incubation with secondary antibody, the immunoblotted proteins were visualized using the ECL Western blotting detection reagent according to the manufacturer's instructions.

Primary antibodies:

rabbit anti PARP (Cell Signaling #9542, 1:1000)
rabbit anti beta Actin (Cell Signaling #4967, 1:3000)
goat anti rabbit IgG , HRP conjugated

Secondary antibody:
goat anti rabbit IgG , HRP conjugated (Dako #P0448, 1:1000)

*Determination of cleaved PARP in cell lysates by MSD Apoptosis Panel*

Cells lysates were prepared according to the Meso Scale Discovery cell lysis protocol. 20 μg of clarified lysate was analyzed in duplicates with MSD Apoptosis Panel Whole Cell Lysate Kit, measuring cleaved PARP signals, according to the manufacturer's instructions. Plates were read on the SECTOR Imager 6000 plate reader.

**RESULTS**

**EFFECT ON CELL GROWTH OF MCF7ARO BREAST CANCER CELLS**

The inhibitory activity of everolimus, exemestane and IGF Ab 60833, as single agents or in combination, on the proliferation of MCF7aro cells was determined in monolayer cultures. For the combination of everolimus and exemestane, 5 concentrations of each drug were tested both as single agents and combined in a matrix format. For the triple combination, the same layout was applied for everolimus and exemestane, and IGF Ab 60833 was added at a fixed concentration of 10 nM to each sample. Three independent experiments with duplicate determinations were carried out for the dual and for the triple combination.

After 6 days of incubation, everolimus and exemestane as single agents showed a dose-dependent, partial reduction of cell growth with a maximal mean CGI of 47% and 60%, respectively. The combination of everolimus and exemestane was more effective, i.e. almost complete cell growth inhibition (mean CGI of >90%) was achieved at 6 different concentration ratios, with a maximal mean CGI of 103% at the highest concentration used for each inhibitor.

The data from the individual experiments and the mean CGI values are shown in Figure 2.

Addition of a fixed concentration of 10 nM of IGF Ab 60833 to the everolimus/exemestane combination resulted in a further enhancement of the anti-proliferative effect. A pronounced induction of net cell death (mean CGI of >120%) in 13 different concentration ratios, with a maximal mean CGI of 149%, after 6 days of incubation with compounds was observed.

The data from the individual experiments and the mean CGI values are shown in Figure 3.

**EFFECT ON AKT AND S6 PHOSPHORYLATION IN MCF7ARO CELLS**

The effect of IGF Ab 60833 and everolimus, as single agents and in combination, on the phosphorylation of

AKT at serine 473 (pAKT S473) and threonine 308 (pAKT T308), and of S6 (pS6) at serine 235/236 was monitored by Western blot analysis of MCF7aro cell lysates prepared 24 hours post treatment.

Treatment with IGF Ab 60833 only at 100 nM did not result in inhibition of AKT or S6 phosphorylation. Upon treatment with everolimus at 0.32 nM, a reduced pS6 signal was observed, whereas pAKT S473 and T308 levels increased compared to the vehicle (DMSO)-treated control. Combined treatment with 100 nM IGF Ab 60833 and 0.32 nM everolimus further reduced pS6 levels, i.e. only a weak signal was detectable, and resulted in pAKT S473 and T308 levels comparable to DMSO-treated control (Figure 4).

**INDUCTION OF APOPTOSIS IN MCF7ARO CELLS**

Cleaved PARP as a marker for apoptosis was analysed by Western blotting and MSD Apoptosis Panel in MCF7aro cell lysates prepared after treatment with everolimus, exemestane and IGF Ab 60833, as single agents or in combination, for 72 h. Levels of cleaved PARP after treatment with the single agents or the combination of everolimus and exemestane were comparable to or only marginally higher than the level in the vehicle-treated control. Treatment with the triple combination of everolimus, exemestane and IGF Ab 60833 resulted in a strong induction of cleaved PARP (Figure 5).

**DISCUSSION**

Despite the fact that rapamycin analogues such as everolimus have shown clinical activity in several cancers, pre-clinical and clinical data suggest that there may be acquired resistance by release of a negative feedback loop when mTORC1 is blocked. Following mTORC1 activation a feedback mechanism results in phosphorylation of IRS-1 which is in turn degraded, thereby inhibiting the signalling pathway. Conversely, when mTORC1 is blocked by rapamycin or a rapalog, the negative feedback mechanism is released and the pathway upstream of mTORC1 is re-activated, resulting in increased AKT phosphorylation.

In mice, rapamycin was found to increase the serum IGF bioactivity suggesting that elevated blood IGF levels may at least in part account for the elevated pAKT levels induced by rapamycin. In models of Ewing's sarcoma, IGF Ab 60833 was shown to inhibit the rapamycin-induced increase in pAKT. Together, these findings suggest that the pAKT elevation upon rapamycin treatment was due to enhanced IGF ligand driven signalling. *In vivo,* the combination of IGF Ab 60833 and rapamycin displayed stronger anti-tumour activity than either single agent alone.

These preclinical studies demonstrated that combination of a rapalog with IGF Ab 60833 leads to a more sustained inhibition of the IGF-1R pathway and can improve efficacy. These data provide a sound rationale for assessing the combination of IGF Ab 60833 with everolimus in estrogen positive breast cancer. Everolimus has recently been approved in combination with the aromatase inhibitor exemestane in this indication.

In the current study, the cellular activity of the triple combination, everolimus, exemestane and IGF Ab 60833, was tested in a human hormone receptor positive breast cancer cell line engineered to express the human aromatase protein (MCF7aro). Under serum-starved conditions, growth of these cells is supported by estrogen which is intracellularly synthesized by the aromatase from androgen supplemented in the growth medium. Using this model, the anti-proliferative activity of aromatase inhibitors can be assessed *in vitro.*

Treatment of MCF7aro cells with the triple combination resulted in increased cell growth inhibition compared to the dual combination everolimus and exemestane. IGF Ab 60833 reversed everolimus-induced AKT phosphorylation, and the combination led to pronounced inhibition of the IGF-1R signalling pathway. Whereas no cell death was observed after concomitant treatment with everolimus and exemestane, addition of IGF Ab 60833 to the dual combination resulted in induction of apoptosis.

**CONCLUSION**

This study has demonstrated that addition of IGF Ab 60833 to the combination of everolimus and exemestane, which is the current standard-of-care treatment in hormone receptor positive breast cancer, leads to improved anti-neoplastic activity *in vitro.*

## Claims

1. An insulin-like growth factor (IGF) receptor antagonist for use in the treatment of patients with breast cancer in combination with exemestane and everolimus, wherein the IGF receptor antagonist is an antibody against both IGF-1 and IGF-2 and having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy

chain variable region of SEQ ID NO: 43 and a light chain variable region of SEQ ID NO: 44, or an antibody having a heavy chain of SEQ ID NO: 19 and a light chain of SEQ ID NO: 20, or an antibody having a heavy chain of SEQ ID NO: 29 and a light chain of SEQ ID NO: 30, or an antibody having a heavy chain of SEQ ID NO: 39 and a light chain of SEQ ID NO: 40.

**2.** The IGF receptor antagonist for use of claim 1 wherein the breast cancer is locally advanced or metastatic breast cancer.

**3.** The IGF receptor antagonist for use of claim 1 or 2 wherein the antibody has a heavy chain of SEQ ID NO: 39 and a light chain of SEQ ID NO: 40.

**4.** The IGF receptor antagonist for use of any of claims 1 to 3, wherein the patient to be treated has one or more characteristics selected from the following: the locally advanced or metastatic breast cancer is positive for estrogen receptor (ER) and / or progesterone receptor (PgR); the locally advanced or metastatic breast cancer is negative for HER2; the locally advanced or metastatic breast cancer is refractory to non-steroidal aromatase inhibitor (e.g. letrozole and/or anastrozole).

**5.** The IGF receptor antagonist for use of claim 4 wherein the patient to be treated has the following characteristics: the locally advanced or metastatic breast cancer is positive for estrogen receptor (ER) and / or progesterone receptor (PgR); and the locally advanced or metastatic breast cancer is also negative for HER2; and the locally advanced or metastatic breast cancer is also refractory to non-steroidal aromatase inhibitor (e.g. letrozole and/or anastrozole).

**6.** The IGF receptor antagonist for use of any one of claims 1 to 5 wherein the patient is a postmenopausal woman.

**7.** The IGF receptor antagonist for use of any one of claims 1 to 5 wherein the patient does not show extensive symptomatic visceral disease.

**8.** Exemestane and everolimus for use in the treatment of patients with breast cancer in combination with an insulin-like growth factor (IGF) receptor antagonist, wherein the IGF receptor antagonist is an antibody against both IGF-1 and IGF-2, and having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy chain variable region of SEQ ID NO: 43 and a light chain variable region of SEQ ID NO: 44, or an antibody having a heavy chain of SEQ ID NO: 19 and a light chain of SEQ ID NO: 20, or an antibody having a heavy chain of SEQ ID NO: 29 and a light chain of SEQ ID NO: 30, or an antibody having a heavy chain of SEQ ID NO: 39 and a light chain of SEQ ID NO: 40.

**9.** The exemestane and everolimus for use of claim 8 wherein the breast cancer is locally advanced or metastatic breast cancer.

**10.** The exemestane and everolimus for use of claim 8 or 9 wherein the patient to be treated has one or more characteristics selected from the following: the locally advanced or metastatic breast cancer is positive for estrogen receptor (ER) and / or progesterone receptor (PgR); the locally advanced or metastatic breast cancer is negative for HER2; the locally advanced or metastatic breast cancer is refractory to non-steroidal aromatase inhibitor (e.g. letrozole and/or anastrozole).

**11.** The exemestane and everolimus for use of claim 10 wherein the patient to be treated has the following characteristics: the locally advanced or metastatic breast cancer is positive for estrogen receptor (ER) and / or progesterone receptor (PgR); and the locally advanced or metastatic breast cancer is also negative for HER2; and the locally advanced

or metastatic breast cancer is also refractory to non-steroidal aromatase inhibitor (e.g. letrozole and/or anastrozole).

**12.** Use of an IGF receptor antagonist for the manufacture of a medicament for the treatment of breast cancer, wherein the IGF receptor antagonist is to be used in combination with exemestane and everolimus and the IGF receptor antagonist is an antibody against both IGF-1 and IGF-2 and having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy chain variable region of SEQ ID NO: 43 and a light chain variable region of SEQ ID NO: 44, or an antibody having a heavy chain of SEQ ID NO: 19 and a light chain of SEQ ID NO: 20, or an antibody having a heavy chain of SEQ ID NO: 29 and a light chain of SEQ ID NO: 30, or an antibody having a heavy chain of SEQ ID NO: 39 and a light chain of SEQ ID NO: 40.

**13.** Use of exemestane and everolimus for the manufacture of a medicament for the treatment of breast cancer, wherein the exemestane and everolimus is to be used in combination with an IGF receptor antagonist and the IGF receptor antagonist is an antibody against both IGF-1 and IGF-2 and having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy chain variable region of SEQ ID NO: 43 and a light chain variable region of SEQ ID NO: 44, or an antibody having a heavy chain of SEQ ID NO: 19 and a light chain of SEQ ID NO: 20, or an antibody having a heavy chain of SEQ ID NO: 29 and a light chain of SEQ ID NO: 30, or an antibody having a heavy chain of SEQ ID NO: 39 and a light chain of SEQ ID NO: 40.

**14.** Pharmaceutical composition, comprising an IGF receptor antagonist and an exemestane and everolimus, together with a pharmaceutically acceptable carrier, wherein the IGF receptor antagonist is an antibody against both IGF-1 and IGF-2 and having heavy chain complementary determining regions of SEQ ID NO: 11 (HCDR1), SEQ ID NO: 12 (HCDR2), and SEQ ID NO: 13 (HCDR3) and light chain determining regions of SEQ ID NO: 14 (LCDR1), SEQ ID NO: 15 (LCDR2), and SEQ ID NO: 16 (LCDR3), or an antibody having heavy chain complementary determining regions of SEQ ID NO: 21 (HCDR1), SEQ ID NO: 22 (HCDR2), and SEQ ID NO: 23 (HCDR3) and light chain determining regions of SEQ ID NO: 24 (LCDR1), SEQ ID NO: 25 (LCDR2), and SEQ ID NO: 26 (LCDR3), or an antibody having heavy chain complementary determining regions of SEQ ID NO: 31 (HCDR1), SEQ ID NO: 32 (HCDR2), and SEQ ID NO: 33 (HCDR3) and light chain determining regions of SEQ ID NO: 34 (LCDR1), SEQ ID NO: 35 (LCDR2), and SEQ ID NO: 36 (LCDR3), or an antibody having a heavy chain variable region of SEQ ID NO: 17 and a light chain variable region of SEQ ID NO: 18, or an antibody having a heavy chain variable region of SEQ ID NO: 27 and a light chain variable region of SEQ ID NO: 28, or an antibody having a heavy chain variable region of SEQ ID NO: 37 and a light chain variable region of SEQ ID NO: 38, or an antibody having a heavy chain variable region of SEQ ID NO: 41 and a light chain variable region of SEQ ID NO: 42, or an antibody having a heavy chain variable region of SEQ ID NO: 43 and a light chain variable region of SEQ ID NO: 44, or an antibody having a heavy chain of SEQ ID NO: 19 and a light chain of SEQ ID NO: 20, or an antibody having a heavy chain of SEQ ID NO: 29 and a light chain of SEQ ID NO: 30, or an antibody having a heavy chain of SEQ ID NO: 39 and a light chain of SEQ ID NO: 40.

## Patentansprüche

1. Insulinähnlicher Wachstumsfaktor (IGF)-Rezeptor-Antagonist zur Verwendung bei der Behandlung von Patienten mit Brustkrebs in Kombination mit Exemestan und Everolimus, wobei der IGF-Rezeptor-Antagonist ein Antikörper sowohl gegen IGF-1 als auch gegen IGF-2 und mit komplementaritätsbestimmenden Regionen der schweren Kette von SEQ ID NO: 11 (HCDR1), SEQ ID NO: 12 (HCDR2) und SEQ ID NO: 13 (HCDR3) und bestimmenden Regionen der leichten Kette von SEQ ID NO: 14 (LCDR1), SEQ ID NO: 15 (LCDR2) und SEQ ID NO: 16 (LCDR3), oder ein Antikörper mit komplementaritätsbestimmenden Regionen der schweren Kette von SEQ ID NO: 21 (HCDR1), SEQ ID NO: 22 (HCDR2) und SEQ ID NO: 23 (HCDR3) und bestimmenden Regionen der leichten Kette von SEQ ID NO: 24 (LCDR1), SEQ ID NO: 25 (LCDR2) und SEQ ID NO: 26 (LCDR3), oder ein Antikörper mit komplementaritätsbestimmenden Regionen der schweren Kette von SEQ ID NO: 31 (HCDR1), SEQ ID NO: 32 (HCDR2) und SEQ ID NO: 33 (HCDR3) und bestimmenden Regionen der leichten Kette von SEQ ID NO: 34 (LCDR1), SEQ ID NO: 35 (LCDR2) und SEQ ID NO: 36 (LCDR3), oder ein Antikörper mit einer variablen Region der schweren Kette von SEQ ID NO: 17 und einer variablen Region der leichten Kette von SEQ ID NO: 18, oder ein Antikörper mit einer variablen Region der schweren Kette von SEQ ID NO: 27 und einer variablen Region der leichten Kette von SEQ ID NO: 28, oder ein Antikörper mit einer variablen Region der schweren Kette von SEQ ID NO: 37 und einer variablen Region der leichten Kette von SEQ ID NO: 38, oder ein Antikörper mit einer variablen Region der schweren Kette von SEQ ID NO: 41 und einer variablen Region der leichten Kette von SEQ ID NO: 42, oder ein Antikörper mit einer variablen Region der schweren Kette von SEQ ID NO: 43 und einer variablen Region der leichten Kette von SEQ ID NO: 44, oder ein Antikörper mit einer schweren Kette von SEQ ID NO: 19 und einer leichten Kette von SEQ ID NO: 20, oder ein Antikörper mit einer schweren Kette von SEQ ID NO: 29 und einer leichten Kette von SEQ ID NO: 30, oder ein Antikörper mit einer schweren Kette von SEQ ID NO: 39 und einer leichten Kette von SEQ ID NO: 40 ist.

2. IGF-Rezeptor-Antagonist zur Verwendung nach Anspruch 1, wobei der Brustkrebs lokal fortgeschrittener oder metastatischer Brustkrebs ist.

3. IGF-Rezeptor-Antagonist zur Verwendung nach Anspruch 1 oder 2, wobei der Antikörper eine schwere Kette von SEQ ID NO: 39 und eine leichte Kette von SEQ ID NO: 40 aufweist.

4. IGF-Rezeptor-Antagonist zur Verwendung nach irgendeinem der Ansprüche 1 bis 3, wobei der zu behandelnde Patient eine oder mehrere Eigenschaften aufweist, die aus Folgenden ausgewählt sind: der lokal fortgeschrittene oder metastatische Brustkrebs ist positiv für Östrogenrezeptor (ER) und/oder Progesteronrezeptor (PgR); der lokal fortgeschrittene oder metastatische Brustkrebs ist negativ für HER2; der lokal fortgeschrittene oder metastatische Brustkrebs ist widerstandfähig gegen nichtsteroidalen Aromatase-Inhibitor (z.B. Letrozol und/oder Anastrozol).

5. IGF-Rezeptor-Antagonist zur Verwendung nach Anspruch 4, wobei der zu behandelnde Patient die folgenden Eigenschaften aufweist: der lokal fortgeschrittene oder metastatische Brustkrebs ist positiv für Östrogenrezeptor (ER) und/oder Progesteronrezeptor (PgR); und der lokal fortgeschrittene oder metastatische Brustkrebs ist auch negativ für HER2; und der lokal fortgeschrittene oder metastatische Brustkrebs ist auch widerstandfähig gegen nichtsteroidalen Aromatase-Inhibitor (z.B. Letrozol und/oder Anastrozol).

6. IGF-Rezeptor-Antagonist zur Verwendung nach irgendeinem der Ansprüche 1 bis 5, wobei der Patient eine postmenopausale Frau ist.

7. IGF-Rezeptor-Antagonist zur Verwendung nach irgendeinem der Ansprüche 1 bis 5, wobei der Patient keine umfangreiche symptomatische viszerale Erkrankung zeigt.

8. Exemestan und Everolimus zur Verwendung bei der Behandlung von Patienten mit Brustkrebs in Kombination mit einem insulinähnlichen Wachstumsfaktor (IGF)-Rezeptor-Antagonisten, wobei der IGF-Rezeptor-Antagonist ein Antikörper sowohl gegen IGF-1 als auch gegen IGF-2 und mit komplementaritätsbestimmenden Regionen der schweren Kette von SEQ ID NO: 11 (HCDR1), SEQ ID NO: 12 (HCDR2) und SEQ ID NO: 13 (HCDR3) und bestimmenden Regionen der leichten (LCDR3), oder ein Antikörper mit komplementaritätsbestimmenden Regionen der schweren Kette von SEQ ID NO: 21 (HCDR1), SEQ ID NO: 22 (HCDR2) und SEQ ID NO: 23 (HCDR3) und bestimmenden Regionen der leichten Kette von SEQ ID NO: 24 (LCDR1), SEQ ID NO: 25 (LCDR2) und SEQ ID NO: 26 (LCDR3), oder ein Antikörper mit komplementaritätsbestimmenden Regionen der schweren Kette von SEQ ID NO: 31 (HCDR1), SEQ ID NO: 32 (HCDR2) und SEQ ID NO: 33 (HCDR3) und bestimmenden Regionen der leichten Kette von SEQ ID NO: 34 (LCDR1), SEQ ID NO: 35 (LCDR2) und SEQ ID NO: 36 (LCDR3), oder ein Antikörper mit einer variablen Region der schweren Kette von SEQ ID NO: 17 und einer variablen Region der leichten Kette

von SEQ ID NO: 18, oder ein Antikörper mit einer variablen Region der schweren Kette von SEQ ID NO: 27 und einer variablen Region der leichten Kette von SEQ ID NO: 28, oder ein Antikörper mit einer variablen Region der schweren Kette von SEQ ID NO: 37 und einer variablen Region der leichten Kette von SEQ ID NO: 38, oder ein Antikörper mit einer variablen Region der schweren Kette von SEQ ID NO: 41 und einer variablen Region der leichten Kette von SEQ ID NO: 42, oder ein Antikörper mit einer variablen Region der schweren Kette von SEQ ID NO: 43 und einer variablen Region der leichten Kette von SEQ ID NO: 44, oder ein Antikörper mit einer schweren Kette von SEQ ID NO: 19 und einer leichten Kette von SEQ ID NO: 20, oder ein Antikörper mit einer schweren Kette von SEQ ID NO: 29 und einer leichten Kette von SEQ ID NO: 30, oder ein Antikörper mit einer schweren Kette von SEQ ID NO: 39 und einer leichten Kette von SEQ ID NO: 40 ist.

**9.** Exemestan und Everolimus zur Verwendung nach Anspruch 8, wobei der Brustkrebs lokal fortgeschrittener oder metastatischer Brustkrebs ist.

**10.** Exemestan und Everolimus zur Verwendung nach Anspruch 8 oder 9, wobei der zu behandelnde Patient eine oder mehrere Eigenschaften aufweist, die aus Folgenden ausgewählt sind: der lokal fortgeschrittene oder metastatische Brustkrebs ist positiv für Östrogenrezeptor (ER) und/oder Progesteronrezeptor (PgR); der lokal fortgeschrittene oder metastatische Brustkrebs ist negativ für HER2; der lokal fortgeschrittene oder metastatische Brustkrebs ist widerstandfähig gegen nichtsteroidalen Aromatase-Inhibitor (z.B. Letrozol und/oder Anastrozol).

**11.** Exemestan und Everolimus zur Verwendung nach Anspruch 10, wobei der zu behandelnde Patient die folgenden Eigenschaften aufweist: der lokal fortgeschrittene oder metastatische Brustkrebs ist positiv für Östrogenrezeptor (ER) und/oder Progesteronrezeptor (PgR); und der lokal fortgeschrittene oder metastatische Brustkrebs ist auch negativ für HER2; und der lokal fortgeschrittene oder metastatische Brustkrebs ist auch widerstandfähig gegen nichtsteroidalen Aromatase-Inhibitor (z.B. Letrozol und/oder Anastrozol).

**12.** Verwendung eines IGF-Rezeptor-Antagonisten zur Herstellung eine Medikaments zur Behandlung von Brustkrebs, wobei der IGF-Rezeptor-Antagonist in Kombination mit Exemestan und Everolimus zu verwenden ist und der IGF-Rezeptor-Antagonist ein Antikörper sowohl gegen IGF-1 als auch gegen IGF-2 und mit komplementaritätsbestimmenden Regionen der schweren Kette von SEQ ID NO: 11 (HCDR1), SEQ ID NO: 12 (HCDR2) und SEQ ID NO: 13 (HCDR3) und bestimmenden Regionen der leichten Kette von SEQ ID NO: 14 (LCDR1), SEQ ID NO: 15 (LCDR2) und SEQ ID NO: 16 (LCDR3), oder ein Antikörper mit komplementaritätsbestimmenden Regionen der schweren Kette von SEQ ID NO: 21 (HCDR1), SEQ ID NO: 22 (HCDR2) und SEQ ID NO: 23 (HCDR3) und bestimmenden Regionen der leichten Kette von SEQ ID NO: 24 (LCDR1), SEQ ID NO: 25 (LCDR2) und SEQ ID NO: 26 (LCDR3), oder ein Antikörper mit komplementaritätsbestimmenden Regionen der schweren Kette von SEQ ID NO: 31 (HCDR1), SEQ ID NO: 32 (HCDR2) und SEQ ID NO: 33 (HCDR3) und bestimmenden Regionen der leichten Kette von SEQ ID NO: 34 (LCDR1), SEQ ID NO: 35 (LCDR2) und SEQ ID NO: 36 (LCDR3), oder ein Antikörper mit einer variablen Region der schweren Kette von SEQ ID NO: 17 und einer variablen Region der leichten Kette von SEQ ID NO: 18, oder ein Antikörper mit einer variablen Region der schweren Kette von SEQ ID NO: 27 und einer variablen Region der leichten Kette von SEQ ID NO: 28, oder ein Antikörper mit einer variablen Region der schweren Kette von SEQ ID NO: 37 und einer variablen Region der leichten Kette von SEQ ID NO: 38, oder ein Antikörper mit einer variablen Region der schweren Kette von SEQ ID NO: 41 und einer variablen Region der leichten Kette von SEQ ID NO: 42, oder ein Antikörper mit einer variablen Region der schweren Kette von SEQ ID NO: 43 und einer variablen Region der leichten Kette von SEQ ID NO: 44, oder ein Antikörper mit einer schweren Kette von SEQ ID NO: 19 und einer leichten Kette von SEQ ID NO: 20, oder ein Antikörper mit einer schweren Kette von SEQ ID NO: 29 und einer leichten Kette von SEQ ID NO: 30, oder ein Antikörper mit einer schweren Kette von SEQ ID NO: 39 und einer leichten Kette von SEQ ID NO: 40 ist.

**13.** Verwendung von Exemestan und Everolimus zur Herstellung eine Medikaments zur Behandlung von Brustkrebs, wobei das Exemestan und Everolimus in Kombination mit einem IGF-Rezeptor-Antagonisten zu verwenden ist und der IGF-Rezeptor-Antagonist ein Antikörper sowohl gegen IGF-1 als auch gegen IGF-2 und mit komplementaritätsbestimmenden Regionen der schweren Kette von SEQ ID NO: 11 (HCDR1), SEQ ID NO: 12 (HCDR2) und SEQ ID NO: 13 (HCDR3) und bestimmenden Regionen der leichten Kette von SEQ ID NO: 14 (LCDR1), SEQ ID NO: 15 (LCDR2) und SEQ ID NO: 16 (LCDR3), oder ein Antikörper mit komplementaritätsbestimmenden Regionen der schweren Kette von SEQ ID NO: 21 (HCDR1), SEQ ID NO: 22 (HCDR2) und SEQ ID NO: 23 (HCDR3) und bestimmenden Regionen der leichten Kette von SEQ ID NO: 24 (LCDR1), SEQ ID NO: 25 (LCDR2) und SEQ ID NO: 26 (LCDR3), oder ein Antikörper mit komplementaritätsbestimmenden Regionen der schweren Kette von SEQ ID NO: 31 (HCDR1), SEQ ID NO: 32 (HCDR2) und SEQ ID NO: 33 (HCDR3) und bestimmenden Regionen der leichten Kette von SEQ ID NO: 34 (LCDR1), SEQ ID NO: 35 (LCDR2) und SEQ ID NO: 36 (LCDR3), oder ein Antikörper

mit einer variablen Region der schweren Kette von SEQ ID NO: 17 und einer variablen Region der leichten Kette von SEQ ID NO: 18, oder ein Antikörper mit einer variablen Region der schweren Kette von SEQ ID NO: 27 und einer variablen Region der leichten Kette von SEQ ID NO: 28, oder ein Antikörper mit einer variablen Region der schweren Kette von SEQ ID NO: 37 und einer variablen Region der leichten Kette von SEQ ID NO: 38, oder ein Antikörper mit einer variablen Region der schweren Kette von SEQ ID NO: 41 und einer variablen Region der leichten Kette von SEQ ID NO: 42, oder ein Antikörper mit einer variablen Region der schweren Kette von SEQ ID NO: 43 und einer variablen Region der leichten Kette von SEQ ID NO: 44, oder ein Antikörper mit einer schweren Kette von SEQ ID NO: 19 und einer leichten Kette von SEQ ID NO: 20, oder ein Antikörper mit einer schweren Kette von SEQ ID NO: 29 und einer leichten Kette von SEQ ID NO: 30, oder ein Antikörper mit einer schweren Kette von SEQ ID NO: 39 und einer leichten Kette von SEQ ID NO: 40 ist.

**14.** Pharmazeutische Zusammensetzung, umfassend einen IGF-Rezeptor-Antagonisten und ein Exemestan und Everolimus, zusammen mit einem pharmazeutisch verträglichen Träger, wobei der IGF-Rezeptor-Antagonist ein Antikörper sowohl gegen IGF-1 als auch gegen IGF-2 und mit komplementaritätsbestimmenden Regionen der schweren Kette von SEQ ID NO: 11 (HCDR1), SEQ ID NO: 12 (HCDR2) und SEQ ID NO: 13 (HCDR3) und bestimmenden Regionen der leichten Kette von SEQ ID NO: 14 (LCDR1), SEQ ID NO: 15 (LCDR2) und SEQ ID NO: 16 (LCDR3), oder ein Antikörper mit komplementaritätsbestimmenden Regionen der schweren Kette von SEQ ID NO: 21 (HCDR1), SEQ ID NO: 22 (HCDR2) und SEQ ID NO: 23 (HCDR3) und bestimmenden Regionen der leichten Kette von SEQ ID NO: 24 (LCDR1), SEQ ID NO: 25 (LCDR2) und SEQ ID NO: 26 (LCDR3), oder ein Antikörper mit komplementaritätsbestimmenden Regionen der schweren Kette von SEQ ID NO: 31 (HCDR1), SEQ ID NO: 32 (HCDR2) und SEQ ID NO: 33 (HCDR3) und bestimmenden Regionen der leichten Kette von SEQ ID NO: 34 (LCDR1), SEQ ID NO: 35 (LCDR2) und SEQ ID NO: 36 (LCDR3), oder ein Antikörper mit einer variablen Region der schweren Kette von SEQ ID NO: 17 und einer variablen Region der leichten Kette von SEQ ID NO: 18, oder ein Antikörper mit einer variablen Region der schweren Kette von SEQ ID NO: 27 und einer variablen Region der leichten Kette von SEQ ID NO: 28, oder ein Antikörper mit einer variablen Region der schweren Kette von SEQ ID NO: 37 und einer variablen Region der leichten Kette von SEQ ID NO: 38, oder ein Antikörper mit einer variablen Region der schweren Kette von SEQ ID NO: 41 und einer variablen Region der leichten Kette von SEQ ID NO: 42, oder ein Antikörper mit einer variablen Region der schweren Kette von SEQ ID NO: 43 und einer variablen Region der leichten Kette von SEQ ID NO: 44, oder ein Antikörper mit einer schweren Kette von SEQ ID NO: 19 und einer leichten Kette von SEQ ID NO: 20, oder ein Antikörper mit einer schweren Kette von SEQ ID NO: 29 und einer leichten Kette von SEQ ID NO: 30, oder ein Antikörper mit einer schweren Kette von SEQ ID NO: 39 und einer leichten Kette von SEQ ID NO: 40 ist.

## Revendications

**1.** Antagoniste du récepteur du facteur de croissance de type insuline (IGF) pour son utilisation dans le traitement de patientes atteintes de cancer du sein en combinaison avec l'exémestane et l'évérolimus, dans lequel l'antagoniste du récepteur à l'IGF est un anticorps contre à la fois l'IGF-1 et l'IGF-2 et ayant des régions déterminant la complémentarité de chaîne lourde de SEQ ID NO : 11 (HCDR1), SEQ ID NO : 12 (HCDR2), et SEQ ID NO : 13 (HCDR3) et des régions déterminant la complémentarité de chaîne légère de SEQ ID NO : 14 (LCDR1), SEQ ID NO : 15 (LCDR2), et SEQ ID NO : 16 (LCDR3), ou un anticorps ayant des régions déterminant la complémentarité de chaîne lourde de SEQ ID NO : 21 (HCDR1), SEQ ID NO : 22 (HCDR2), et SEQ ID NO : 23 (HCDR3) et des régions déterminant la complémentarité de chaîne légère de SEQ ID NO : 24 (LCDR1), SEQ ID NO : 25 (LCDR2), et SEQ ID NO : 26 (LCDR3), ou un anticorps ayant des régions déterminant la complémentarité de chaîne lourde de SEQ ID NO : 31 (HCDR1), SEQ ID NO : 32 (HCDR2), et SEQ ID NO : 33 (HCDR3) et des régions déterminant la complémentarité de chaîne légère de SEQ ID NO : 34 (LCDR1), SEQ ID NO : 35 (LCDR2), et SEQ ID NO : 36 (LCDR3), ou un anticorps ayant une région variable de chaîne lourde de SEQ ID NO : 17 et une région variable de chaîne légère de SEQ ID NO : 18, ou un anticorps ayant une région variable de chaîne lourde de SEQ ID NO : 27 et une région variable de chaîne légère de SEQ ID NO : 28, ou un anticorps ayant une région variable de chaîne lourde de SEQ ID NO : 37 et une région variable de chaîne légère de SEQ ID NO : 38, ou un anticorps ayant une région variable de chaîne lourde de SEQ ID NO : 41 et une région variable de chaîne légère de SEQ ID NO : 42, ou un anticorps ayant une région variable de chaîne lourde de SEQ ID NO : 43 et une région variable de chaîne légère de SEQ ID NO : 44, ou un anticorps ayant une chaîne lourde de SEQ ID NO : 19 et une chaîne légère de SEQ ID NO : 20, ou un anticorps ayant une chaîne lourde de SEQ ID NO : 29 et une chaîne légère de SEQ ID NO : 30, ou un anticorps ayant une chaîne lourde de SEQ ID NO : 39 et une chaîne légère de SEQ ID NO : 40.

**2.** Antagoniste du récepteur à l'IGF pour son utilisation selon la revendication 1 dans lequel le cancer du sein est un

cancer du sein localement avancé ou métastatique.

3. Antagoniste du récepteur à l'IGF pour son utilisation selon la revendication 1 ou 2 dans lequel l'anticorps a une chaîne lourde de SEQ ID NO : 39 et une chaîne légère de SEQ ID NO : 40.

4. Antagoniste du récepteur à l'IGF pour son utilisation selon l'une quelconque des revendications 1 à 3, dans lequel la patiente à traiter présente une ou plusieurs caractéristiques sélectionnées parmi les suivantes : le cancer du sein localement avancé ou métastatique est positif au récepteur aux oestrogènes (ER) et/ou au récepteur à la progestérone (PgR) ; le cancer du sein localement avancé ou métastatique est négatif pour HER2 ; le cancer du sein localement avancé ou métastatique est réfractaire à un inhibiteur de l'aromatase non stéroïdien (par exemple, le létrozole et/ou l'anastrozole).

5. Antagoniste du récepteur à l'IGF pour son utilisation selon la revendication 4 dans lequel la patiente à traiter présente les caractéristiques suivantes : le cancer du sein localement avancé ou métastatique est positif au récepteur aux oestrogènes (ER) et/ou au récepteur à la progestérone (PgR) ; et le cancer du sein localement avancé ou métastatique est également négatif pour HER2 ; et le cancer du sein localement avancé ou métastatique est également réfractaire à un inhibiteur de l'aromatase non stéroïdien (par exemple, le létrozole et/ou l'anastrozole).

6. Antagoniste du récepteur à l'IGF pour son utilisation selon l'une quelconque des revendications 1 à 5 dans lequel la patiente est une femme post-ménopausée.

7. Antagoniste du récepteur à l'IGF pour son utilisation selon l'une quelconque des revendications 1 à 5 dans lequel la patiente ne présente pas de maladie viscérale symptomatique extensive.

8. Exémestane et évérolimus pour leur utilisation dans le traitement de patientes atteintes de cancer du sein en combinaison avec un antagoniste du récepteur du facteur de croissance de type insuline (IGF), dans lequel l'antagoniste du récepteur à l'IGF est un anticorps contre à la fois l'IGF-1 et l'IGF-2, et ayant des régions déterminant la complémentarité de chaîne lourde de SEQ ID NO : 11 (HCDR1), SEQ ID NO : 12 (HCDR2), et SEQ ID NO : 13 (HCDR3) et des régions déterminant la complémentarité de chaîne légère de SEQ ID NO : 14 (LCDR1), SEQ ID NO : 15 (LCDR2), et SEQ ID NO : 16 (LCDR3), ou un anticorps ayant des régions déterminant la complémentarité de chaîne lourde de SEQ ID NO : 21 (HCDR1), SEQ ID NO : 22 (HCDR2), et SEQ ID NO : 23 (HCDR3) et des régions déterminant la complémentarité de chaîne légère de SEQ ID NO : 24 (LCDR1), SEQ ID NO : 25 (LCDR2), et SEQ ID NO : 26 (LCDR3), ou un anticorps ayant des régions déterminant la complémentarité de chaîne lourde de SEQ ID NO : 31 (HCDR1), SEQ ID NO : 32 (HCDR2), et SEQ ID NO : 33 (HCDR3) et des régions déterminant la complémentarité de chaîne légère de SEQ ID NO : 34 (LCDR1), SEQ ID NO : 35 (LCDR2), et SEQ ID NO : 36 (LCDR3), ou un anticorps ayant une région variable de chaîne lourde de SEQ ID NO : 17 et une région variable de chaîne légère de SEQ ID NO : 18, ou un anticorps ayant une région variable de chaîne lourde de SEQ ID NO : 27 et une région variable de chaîne légère de SEQ ID NO : 28, ou un anticorps ayant une région variable de chaîne lourde de SEQ ID NO : 37 et une région variable de chaîne légère de SEQ ID NO : 38, ou un anticorps ayant une région variable de chaîne lourde de SEQ ID NO : 41 et une région variable de chaîne légère de SEQ ID NO : 42, ou un anticorps ayant une région variable de chaîne lourde de SEQ ID NO : 43 et une région variable de chaîne légère de SEQ ID NO : 44, ou un anticorps ayant une chaîne lourde de SEQ ID NO : 19 et une chaîne légère de SEQ ID NO : 20, ou un anticorps ayant une chaîne lourde de SEQ ID NO : 29 et une chaîne légère de SEQ ID NO : 30, ou un anticorps ayant une chaîne lourde de SEQ ID NO : 39 et une chaîne légère de SEQ ID NO : 40.

9. Exémestane et évérolimus pour leur utilisation selon la revendication 8 dans lesquels le cancer du sein est un cancer du sein localement avancé ou métastatique.

10. Exémestane et évérolimus pour leur utilisation selon la revendication 8 ou 9 dans lesquels la patiente à traiter présente une ou plusieurs caractéristiques sélectionnées parmi : le cancer du sein localement avancé ou métastatique est positif au récepteur aux oestrogènes (ER) et/ou au récepteur à la progestérone (PgR) ; le cancer du sein localement avancé ou métastatique est négatif pour HER2 ; le cancer du sein localement avancé ou métastatique est réfractaire à un inhibiteur de l'aromatase non stéroïdien (par exemple, le létrozole et/ou l'anastrozole).

11. Exémestane et évérolimus pour leur utilisation selon la revendication 10 dans lesquels la patiente à traiter présente les caractéristiques suivantes : le cancer du sein localement avancé ou métastatique est positif au récepteur aux oestrogènes (ER) et/ou au récepteur à la progestérone (PgR) ; et le cancer du sein localement avancé ou métastatique est également négatif pour HER2 ; et le cancer du sein localement avancé ou métastatique est également

réfractaire à un inhibiteur de l'aromatase non stéroïdien (par exemple, le létrozole et/ou l'anastrozole).

12. Utilisation d'un antagoniste du récepteur à l'IGF pour la fabrication d'un médicament pour le traitement du cancer du sein, dans lequel l'antagoniste du récepteur à l'IGF doit être utilisé en combinaison avec l'exémestane et l'évérolimus et l'antagoniste du récepteur à l'IGF est un anticorps contre à la fois l'IGF-1 et l'IGF-2 et ayant des régions déterminant la complémentarité de chaîne lourde de SEQ ID NO : 11 (HCDR1), SEQ ID NO : 12 (HCDR2), et SEQ ID NO : 13 (HCDR3) et des régions déterminant la complémentarité de chaîne légère de SEQ ID NO : 14 (LCDR1), SEQ ID NO : 15 (LCDR2), et SEQ ID NO : 16 (LCDR3), ou un anticorps ayant des régions déterminant la complémentarité de chaîne lourde de SEQ ID NO : 21 (HCDR1), SEQ ID NO : 22 (HCDR2), et SEQ ID NO : 23 (HCDR3) et des régions déterminant la complémentarité de chaîne légère de SEQ ID NO : 24 (LCDR1), SEQ ID NO : 25 (LCDR2), et SEQ ID NO : 26 (LCDR3), ou un anticorps ayant des régions déterminant la complémentarité de chaîne lourde de SEQ ID NO : 31 (HCDR1), SEQ ID NO : 32 (HCDR2), et SEQ ID NO : 33 (HCDR3) et des régions déterminant la complémentarité de chaîne légère de SEQ ID NO : 34 (LCDR1), SEQ ID NO : 35 (LCDR2), et SEQ ID NO : 36 (LCDR3), ou un anticorps ayant une région variable de chaîne lourde de SEQ ID NO : 17 et une région variable de chaîne légère de SEQ ID NO : 18, ou un anticorps ayant une région variable de chaîne lourde de SEQ ID NO : 27 et une région variable de chaîne légère de SEQ ID NO : 28, ou un anticorps ayant une région variable de chaîne lourde de SEQ ID NO : 37 et une région variable de chaîne légère de SEQ ID NO : 38, ou un anticorps ayant une région variable de chaîne lourde de SEQ ID NO : 41 et une région variable de chaîne légère de SEQ ID NO : 42, ou un anticorps ayant une région variable de chaîne lourde de SEQ ID NO : 43 et une région variable de chaîne légère de SEQ ID NO : 44, ou un anticorps ayant une chaîne lourde de SEQ ID NO : 19 et une chaîne légère de SEQ ID NO : 20, ou un anticorps ayant une chaîne lourde de SEQ ID NO : 29 et une chaîne légère de SEQ ID NO : 30, ou un anticorps ayant une chaîne lourde de SEQ ID NO : 39 et une chaîne légère de SEQ ID NO : 40.

13. Utilisation d'exémestane et d'évérolimus pour la fabrication d'un médicament pour le traitement du cancer du sein, dans lequel l'exémestane et l'évérolimus doivent être utilisés en combinaison avec un antagoniste du récepteur à l'IGF et l'antagoniste du récepteur à l'IGF est un anticorps contre à la fois l'IGF-1 et l'IGF-2 et ayant des régions déterminant la complémentarité de chaîne lourde de SEQ ID NO : 11 (HCDR1), SEQ ID NO : 12 (HCDR2), et SEQ ID NO : 13 (HCDR3) et des régions déterminant la complémentarité de chaîne légère de SEQ ID NO : 14 (LCDR1), SEQ ID NO : 15 (LCDR2), et SEQ ID NO : 16 (LCDR3), ou un anticorps ayant des régions déterminant la complémentarité de chaîne lourde de SEQ ID NO : 21 (HCDR1), SEQ ID NO : 22 (HCDR2), et SEQ ID NO : 23 (HCDR3) et des régions déterminant la complémentarité de chaîne légère de SEQ ID NO : 24 (LCDR1), SEQ ID NO : 25 (LCDR2), et SEQ ID NO : 26 (LCDR3), ou un anticorps ayant des régions déterminant la complémentarité de chaîne lourde de SEQ ID NO : 31 (HCDR1), SEQ ID NO : 32 (HCDR2), et SEQ ID NO : 33 (HCDR3) et des régions déterminant la complémentarité de chaîne légère de SEQ ID NO : 34 (LCDR1), SEQ ID NO : 35 (LCDR2), et SEQ ID NO : 36 (LCDR3), ou un anticorps ayant une région variable de chaîne lourde de SEQ ID NO : 17 et une région variable de chaîne légère de SEQ ID NO : 18, ou un anticorps ayant une région variable de chaîne lourde de SEQ ID NO : 27 et une région variable de chaîne légère de SEQ ID NO : 28, ou un anticorps ayant une région variable de chaîne lourde de SEQ ID NO : 37 et une région variable de chaîne légère de SEQ ID NO : 38, ou un anticorps ayant une région variable de chaîne lourde de SEQ ID NO : 41 et une région variable de chaîne légère de SEQ ID NO : 42, ou un anticorps ayant une région variable de chaîne lourde de SEQ ID NO : 43 et une région variable de chaîne légère de SEQ ID NO : 44, ou un anticorps ayant une chaîne lourde de SEQ ID NO : 19 et une chaîne légère de SEQ ID NO : 20, ou un anticorps ayant une chaîne lourde de SEQ ID NO : 29 et une chaîne légère de SEQ ID NO : 30, ou un anticorps ayant une chaîne lourde de SEQ ID NO : 39 et une chaîne légère de SEQ ID NO : 40.

14. Composition pharmaceutique, comprenant un antagoniste de récepteur à l'IGF et de l'exémestane et de l'évérolimus, conjointement à un excipient pharmaceutiquement acceptable, dans lequel l'antagoniste du récepteur à l'IGF est un anticorps contre à la fois l'IGF-1 et l'IGF-2 et ayant des régions déterminant la complémentarité de chaîne lourde de SEQ ID NO : 11 (HCDR1), SEQ ID NO : 12 (HCDR2), et SEQ ID NO : 13 (HCDR3) et des régions déterminant la complémentarité de chaîne légère de SEQ ID NO : 14 (LCDR1), SEQ ID NO : 15 (LCDR2), et SEQ ID NO : 16 (LCDR3), ou un anticorps ayant des régions déterminant la complémentarité de chaîne lourde de SEQ ID NO : 21 (HCDR1), SEQ ID NO : 22 (HCDR2), et SEQ ID NO : 23 (HCDR3) et des régions déterminant la complémentarité de chaîne légère de SEQ ID NO : 24 (LCDR1), SEQ ID NO : 25 (LCDR2), et SEQ ID NO : 26 (LCDR3), ou un anticorps ayant des régions déterminant la complémentarité de chaîne lourde de SEQ ID NO : 31 (HCDR1), SEQ ID NO : 32 (HCDR2), et SEQ ID NO : 33 (HCDR3) et des régions déterminant la complémentarité de chaîne légère de SEQ ID NO : 34 (LCDR1), SEQ ID NO : 35 (LCDR2), et SEQ ID NO : 36 (LCDR3), ou un anticorps ayant une région variable de chaîne lourde de SEQ ID NO : 17 et une région variable de chaîne légère de SEQ ID NO : 18, ou un anticorps ayant une région variable de chaîne lourde de SEQ ID NO : 27 et une région variable de chaîne légère de SEQ ID NO : 28, ou un anticorps ayant une région variable de chaîne lourde de SEQ ID NO : 37 et une

région variable de chaîne légère de SEQ ID NO : 38, ou un anticorps ayant une région variable de chaîne lourde de SEQ ID NO : 41 et une région variable de chaîne légère de SEQ ID NO : 42, ou un anticorps ayant une région variable de chaîne lourde de SEQ ID NO : 43 et une région variable de chaîne légère de SEQ ID NO : 44, ou un anticorps ayant une chaîne lourde de SEQ ID NO : 19 et une chaîne légère de SEQ ID NO : 20, ou un anticorps ayant une chaîne lourde de SEQ ID NO : 29 et une chaîne légère de SEQ ID NO : 30, ou un anticorps ayant une chaîne lourde de SEQ ID NO : 39 et une chaîne légère de SEQ ID NO : 40.

# Fig. 1

Everolimus [nM] + 10 nM IGF Ab60833

(A)

Double combination: Everolimus + Exemestane

EX00003557 / Exemestane [nmol/l]

| | | | | | | |
|---|---|---|---|---|---|---|
| 200 | 60 | 63 | 78 | 79 | 84 | 87 |
| 40 | 48 | 51 | 69 | 70 | 76 | 79 |
| 8 | 11 | 15 | 41 | 42 | 50 | 54 |
| 1.6 | 1 | 6 | 33 | 34 | 43 | 48 |
| ).32 | 6 | 10 | 36 | 38 | 46 | 51 |
| 0 | | 4 | 32 | 33 | 42 | 47 |
| | 0 | 0.08 | 0.4 | 2 | 10 | 50 |

EX00100386 / Everolimus [nmol/l]

(B)

Triple combination: Everolimus + Exemestane + 60833 (10 nM)

Exemestane / 10 nM mAB [nmol/l]

| | | | | | | |
|---|---|---|---|---|---|---|
| 200 | 103 | 146 | 157 | 160 | 164 | 166 |
| 40 | 101 | 141 | 152 | 156 | 161 | 163 |
| 8 | 72 | 117 | 129 | 134 | 140 | 143 |
| 1.6 | 53 | 104 | 117 | 121 | 126 | 130 |
| 0.32 | 61 | 108 | 121 | 127 | 134 | 138 |
| 0 | | 74 | 90 | 98 | 101 | 106 |
| | 0 | 0.08 | 0.4 | 2 | 10 | 50 |

Everolimus / 10 nM mAB [nmol/l]

# Fig. 2

A

EXPERIMENT 1

| Exemestane [nM] | | | | | | |
|---|---|---|---|---|---|---|
| 200 | 73 | 85 | 109 | 105 | 115 | 113 |
| 40 | 57 | 87 | 87 | 96 | 104 | 105 |
| 8 | 9 | 29 | 57 | 71 | 76 | 72 |
| 1.6 | 5 | -12 | 29 | 41 | 47 | 53 |
| 0.32 | 5 | 0 | 32 | 46 | 60 | 59 |
| 0 | | -5 | 31 | 45 | 43 | 40 |
| | 0 | 0.08 | 0.4 | 2 | 10 | 50 |

Everolimus [nM]

B

EXPERIMENT 2

| Exemestane [nM] | | | | | | |
|---|---|---|---|---|---|---|
| 200 | 62 | 73 | 89 | 97 | 98 | 110 |
| 40 | 50 | 66 | 78 | 86 | 90 | 91 |
| 8 | 21 | 22 | 44 | 67 | 67 | 71 |
| 1.6 | 2 | 7 | 26 | 40 | 44 | 53 |
| 0.32 | 7 | 8 | 22 | 35 | 42 | 46 |
| 0 | | 0 | 30 | 27 | 37 | 51 |
| | 0 | 0.08 | 0.4 | 2 | 10 | 50 |

Everolimus [nM]

C

EXPERIMENT 3

| Exemestane [nM] | | | | | | |
|---|---|---|---|---|---|---|
| 200 | 45 | 65 | 84 | 79 | 82 | 86 |
| 40 | 39 | 67 | 76 | 77 | 80 | 84 |
| 8 | 3 | 23 | 48 | 62 | 61 | 65 |
| 1.6 | -3 | 18 | 33 | 43 | 42 | 47 |
| 0.32 | 5 | 24 | 40 | 41 | 42 | 56 |
| 0 | | 19 | 35 | 27 | 46 | 50 |
| | 0 | 0.08 | 0.4 | 2 | 10 | 50 |

Everolimus [nM]

D

MEAN OF EXPERIMENT 1-3

| Exemestane [nM] | | | | | | |
|---|---|---|---|---|---|---|
| 200 | 60 | 74 | 94 | 94 | 98 | 103 |
| 40 | 48 | 73 | 80 | 86 | 91 | 93 |
| 8 | 11 | 25 | 49 | 67 | 68 | 69 |
| 1.6 | 1 | 4 | 30 | 41 | 44 | 51 |
| 0.32 | 6 | 10 | 31 | 40 | 48 | 53 |
| 0 | | 4 | 32 | 33 | 42 | 47 |
| | 0 | 0.08 | 0.4 | 2 | 10 | 50 |

Everolimus [nM]

# Fig. 3

A

EXPERIMENT 1

| Exemestane [nM] + 10 nM IGF Ab60833 | | | | | | |
|---|---|---|---|---|---|---|
| 200 | 129 | 147 | 156 | 160 | 160 | 158 |
| 40 | 136 | 152 | 157 | 163 | 165 | 165 |
| 8 | 98 | 138 | 147 | 158 | 156 | 161 |
| 1.6 | 77 | 105 | 123 | 142 | 133 | 144 |
| 0.32 | 72 | 97 | 122 | 132 | 126 | 135 |
| 0 | | 84 | 105 | 126 | 122 | 132 |
| | 0 | 0.08 | 0.4 | 2 | 10 | 50 |

Everolimus [nM] + 10 nM IGF Ab60833

B

EXPERIMENT 2

| Exemestane [nM] + 10 nM IGF | | | | | | |
|---|---|---|---|---|---|---|
| 200 | 95 | 111 | 143 | 144 | 146 | 151 |
| 40 | 88 | 142 | 151 | 148 | 154 | 160 |
| 8 | 62 | 96 | 123 | 123 | 132 | 137 |
| 1.6 | 40 | 79 | 99 | 96 | 108 | 120 |
| 0.32 | 52 | 79 | 89 | 92 | 96 | 100 |
| 0 | | 68 | 85 | 85 | 94 | 101 |
| | 0 | 0.08 | 0.4 | 2 | 10 | 50 |

Everolimus [nM] + 10 nM IGF Ab60833

C

EXPERIMENT 3

| Exemestane [nM] + 10 nM IGF Ab60833 | | | | | | |
|---|---|---|---|---|---|---|
| 200 | 86 | 98 | 118 | 104 | 112 | 118 |
| 40 | 80 | 91 | 98 | 110 | 100 | 120 |
| 8 | 57 | 83 | 94 | 97 | 97 | 99 |
| 1.6 | 42 | 69 | 84 | 88 | 87 | 90 |
| 0.32 | 58 | 71 | 78 | 86 | 89 | 87 |
| 0 | | 71 | 79 | 83 | 88 | 87 |
| | 0 | 0.08 | 0.4 | 2 | 10 | 50 |

Everolimus [nM] + 10 nM IGF Ab60833

D

MEAN OF EXPERIMENT 1-3

| Exemestane [nM] + 10 nM IGF Ab60833 | | | | | | |
|---|---|---|---|---|---|---|
| 200 | 103 | 118 | 139 | 136 | 139 | 142 |
| 40 | 101 | 128 | 135 | 140 | 140 | 149 |
| 8 | 72 | 106 | 121 | 126 | 128 | 132 |
| 1.6 | 53 | 84 | 102 | 108 | 109 | 118 |
| 0.32 | 61 | 82 | 96 | 103 | 104 | 107 |
| 0 | | 74 | 90 | 98 | 101 | 106 |
| | 0 | 0.08 | 0.4 | 2 | 10 | 50 |

Everolimus [nM] + 10 nM IGF Ab60833

Fig. 4

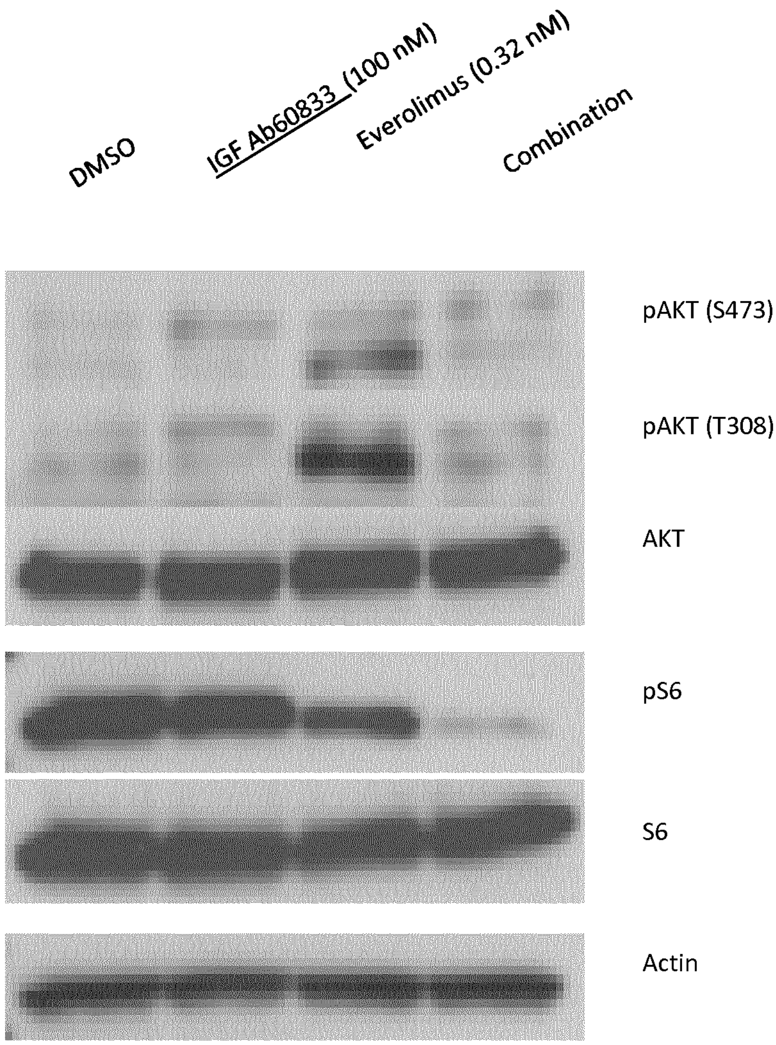
DMSO
IGF Ab60833 (100 nM)
Everolimus (0.32 nM)
Combination
pAKT (S473)
pAKT (T308)
AKT
pS6
S6
Actin

Fig. 5

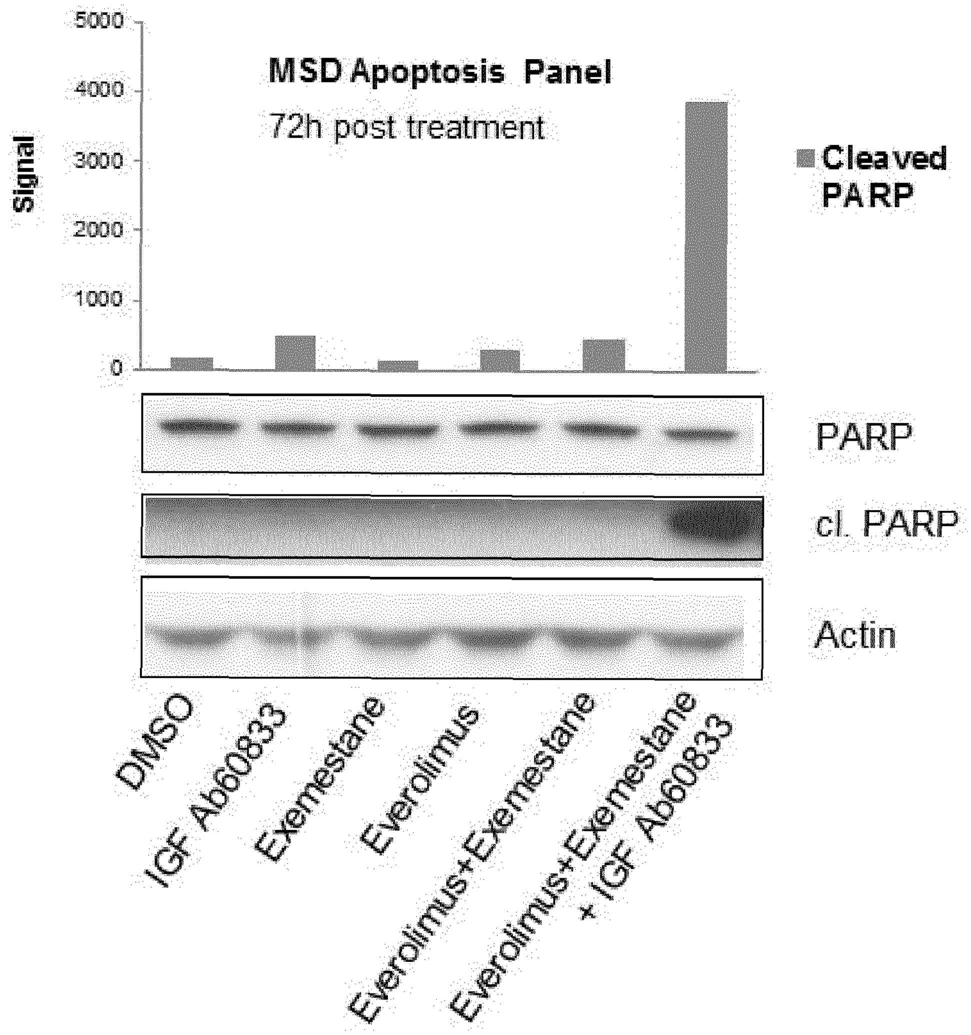

MSD Apoptosis Panel
72h post treatment
Signal
5000
4000
3000
2000
1000
0
Cleaved PARP
PARP
cl. PARP
Actin
DMSO
IGF Ab60833
Exemestane
Everolimus
Everolimus+Exemestane
Everolimus+Exemestane + IGF Ab60833

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013169611 A
- WO 2010066868 A
- WO 200253596 A
- WO 2007070432 A
- WO 2008152422 A
- WO 2008155387 A


### Non-patent literature cited in the description


- **FOROUZANFAR M ; FOREMAN K ; DELOSSANTOS AM ; LOZANO R ; LOPEZ AD ; MURRAY CJL et al.** *Lancet,* 2011, vol. 378, 1461-1484
- **VILLARREAL-GARZA C ; CORTES J ; ANDRE F ; VERMA S.** *Ann Oncol,* 2012, vol. 23 (10), 2526-2535
- **BACHELOT T ; BOURGIER C ; CROPET C ; RAY-COQUARD I ; FERRERO JM ; FREYER G et al.** *J Clin Oncol,* 2012, vol. 30 (22), 2718-2724
- **BASELGA J ; CAMPONE M ; PICCART M ; BURRIS HA ; RUGO HS ; SAHMOUD T et al.** *N Engl J Med,* 2012, vol. 366 (6), 520-529
- **POLLACK et al.** *Nature Rev. Can.,* 2004, vol. 4, 505-518
- **LEROITH D.** *Experimental Diab. Res.,* 2003, vol. 4, 205-212
- **RUBIN et al.** *Lab. Invest.,* 1995, vol. 73, 311-31
- **FRASCA et al.** *Mol. Cell. Biol.,* 1999, vol. 19, 3278-88
- **PANDINI et al.** *J. Biol. Chem.,* 2002, vol. 277, 39684-95
- **JEROME et al.** *End. Rel. Cancer,* 2003, vol. 10, 561-578
- **CULLEN KJ ; YEE D ; SLY WS ; PERDUE J ; HAMPTON B ; LIPPMAN ME ; ROSEN N.** *Cancer Res,* 1990, vol. 50 (1), 48-53
- **YANG Y ; YEE D.** *J Mammary Gland Biol Neoplasia,* 2012, vol. 17 (3/4), 251-261
- **PEYRAT JP ; BONNETERRE J ; BEUSCART R ; DIJANE J ; DEMAILLE A.** *Cancer Res,* 1988, vol. 48 (22), 6429-6433
- **ZARDAVAS, D ; BASALGA J ; PICCART M.** *Nat Rev Clin Oncol.,* April 2013, vol. 10 (4), 191-210
- **CLARKE RB ; HOWELL A ; ANDERSON E.** *Br J Cancer,* 1997, vol. 75 (2), 251-257
- **HAMELERS IHL ; SCHAIK RFMA VAN ; TEEFFELEN HAAM VAN ; SUSSENBACH JS ; STEENBERGH PH.** *Exp Cell Res,* vol. 273, 107-117
- **WISEMAN LR ; JOHNSON MD ; WAKELING AE ; LYKKESFELDT AE ; MAY FEB ; WESTLEY BR.** *Eur J Cancer (A),* 1993, vol. 29 (16), 2256-2264
- **BACHELOT T ; BOURGIER C ; CROPET C ; RAY-COQUARD I ; FERRERO JM ; FREYER G et al.** *J Clin Oncol,* 2012, vol. 30 (22), 2718-2724
- **SUN SY ; ROSENBERG LM ; WANG X ; ZHOU Z ; YUE P ; FU H et al.** *Cancer Res.,* 15 August 2005, vol. 65 (16), 7052-8
- **TABERNERO J ; ROJO F ; CALVO E ; BURRIS H ; JUDSON I ; HAZELL K et al.** *J Clin Oncol,* 2008, vol. 26 (10), 1603-1610
- **WAN X ; HARKAVY B ; SHEN N ; GROHAR P ; HELMAN LJ.** *Oncogene,* 2007, vol. 26, 1932-1940
- **O'REILLY KE ; ROJO F ; SHE QB ; SOLIT D ; MILLS GB ; SMITH D et al.** *Cancer Res,* 2006, vol. 66 (3), 1500-1508
- **HIGGINS MJ ; BASELGA J.** *J Clin Invest,* 2011, vol. 121 (10), 3797-3803

SEQUENCE LISTING

<110> Boehringer Ingelheim International GmbH

<120> IGF Antibody triple combination

<130> P12-0376-PCT

<160> 46

<170> BiSSAP 1.0

<210> 1
<211> 5
<212> PRT
<213> Homo sapiens

<220>
<221> SOURCE
<222> 1..5
<223> /mol_type="protein" /organism="Homo sapiens"

<400> 1

```
Asn Tyr Trp Met His
1               5
```

<210> 2
<211> 17
<212> PRT
<213> Homo sapiens

<220>
<221> SOURCE
<222> 1..17
<223> /mol_type="protein" /organism="Homo sapiens"

<400> 2

```
Gly Ile Ser Gly Trp Ser Ser Trp Thr Tyr Tyr Ala Asp Ser Val Lys
1               5                   10                  15
Gly
```

<210> 3
<211> 13
<212> PRT
<213> Homo sapiens

<220>
<221> SOURCE
<222> 1..13
<223> /mol_type="protein" /organism="Homo sapiens"

<400> 3

```
Phe Gly Ile Asp Ala Tyr Thr Lys Val Tyr Phe Asp Tyr
1               5                   10
```

<210> 4
<211> 11
<212> PRT
<213> Homo sapiens

<220>
<221> SOURCE
<222> 1..11
<223> /mol_type="protein" /organism="Homo sapiens"

<400> 4

```
Ser Gly Asp Asn Ile Pro Leu Lys Tyr Val Ser
1               5                   10
```

<210> 5
<211> 7
<212> PRT
<213> Homo sapiens

<220>
<221> SOURCE
<222> 1..7
<223> /mol_type="protein" /organism="Homo sapiens"

<400> 5

```
Asp Asp Asn Lys Arg Pro Ser
1               5
```

<210> 6
<211> 9
<212> PRT
<213> Homo sapiens

<220>
<221> SOURCE
<222> 1..9
<223> /mol_type="protein" /organism="Homo sapiens"

<400> 6

```
Gln Ser Trp Ala Ser Thr Gly Val Val
1               5
```

<210> 7
<211> 122
<212> PRT
<213> Homo sapiens

<220>
<221> SOURCE
<222> 1..122
<223> /mol_type="protein" /organism="Homo sapiens"

<400> 7

Gln Val Glu Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Trp Met His Trp Val Arg Gln Ala Pro Gly Lys Gly Leu Glu Trp Val
        35                  40                  45
Ser Gly Ile Ser Gly Trp Ser Ser Trp Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95
Ala Arg Phe Gly Ile Asp Ala Tyr Thr Lys Val Tyr Phe Asp Tyr Trp
            100                 105                 110
Gly Gln Gly Thr Leu Val Thr Val Ser Ser
        115                 120

<210> 8
<211> 107
<212> PRT
<213> Homo sapiens

<220>
<221> SOURCE
<222> 1..107
<223> /mol_type="protein" /organism="Homo sapiens"

<400> 8

Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu
65                  70                  75                  80
Asp Glu Ala Asp Tyr Tyr Cys Gln Ser Trp Ala Ser Thr Gly Val Val
                85                  90                  95
Phe Gly Gly Gly Thr Lys Leu Thr Val Leu Gly
            100                 105

<210> 9
<211> 452
<212> PRT
<213> Homo sapiens

<220>
<221> SOURCE
<222> 1..452
<223> /mol_type="protein" /organism="Homo sapiens"

<400> 9

```
Gln Val Glu Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Trp Met His Trp Val Arg Gln Ala Pro Gly Lys Gly Leu Glu Trp Val
        35                  40                  45
Ser Gly Ile Ser Gly Trp Ser Ser Trp Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Phe Gly Ile Asp Ala Tyr Thr Lys Val Tyr Phe Asp Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Ser Ser Lys Ser Thr Ser Gly Gly Thr


    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450
```

<210> 10
<211> 212
<212> PRT
<213> Homo sapiens

<220>
<221> SOURCE
<222> 1..212
<223> /mol_type="protein" /organism="Homo sapiens"

<400> 10

```
Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu

65                  70                  75                  80
Asp Glu Ala Asp Tyr Tyr Cys Gln Ser Trp Ala Ser Thr Gly Val Val
                85                  90                  95
Phe Gly Gly Gly Thr Lys Leu Thr Val Leu Gly Gln Pro Lys Ala Ala
            100                 105                 110
Pro Ser Val Thr Leu Phe Pro Pro Ser Ser Glu Glu Leu Gln Ala Asn
        115                 120                 125
Lys Ala Thr Leu Val Cys Leu Ile Ser Asp Phe Tyr Pro Gly Ala Val
    130                 135                 140
Thr Val Ala Trp Lys Gly Asp Ser Ser Pro Val Lys Ala Gly Val Glu
145                 150                 155                 160
Thr Thr Thr Pro Ser Lys Gln Ser Asn Asn Lys Tyr Ala Ala Ser Ser
                165                 170                 175
Tyr Leu Ser Leu Thr Pro Glu Gln Trp Lys Ser His Arg Ser Tyr Ser
            180                 185                 190
Cys Gln Val Thr His Glu Gly Ser Thr Val Glu Lys Thr Val Ala Pro
        195                 200                 205
Thr Glu Cys Ser
    210
```

<210> 11
<211> 5
<212> PRT
<213> Homo sapiens

<220>
<221> SOURCE
<222> 1..5
<223> /mol_type="protein" /organism="Homo sapiens"

<400> 11

```
Asn Tyr Trp Met His
1               5
```

<210> 12
<211> 17

<212> PRT
<213> Homo sapiens

<220>
<221> SOURCE
<222> 1..17
<223> /mol_type="protein" /organism="Homo sapiens"

<400> 12

```
Gly Ile Ser Gly Trp Ser Ser Trp Thr Tyr Tyr Ala Asp Ser Val Lys
1               5                   10                  15
Gly
```

<210> 13
<211> 13
<212> PRT
<213> Homo sapiens

<220>
<221> SOURCE
<222> 1..13
<223> /mol_type="protein" /organism="Homo sapiens"

<400> 13

```
Phe Gly Ile Asp Ala Tyr Thr Lys Val Tyr Phe Asp Tyr
1               5                   10
```

<210> 14
<211> 11
<212> PRT
<213> Homo sapiens

<220>
<221> SOURCE
<222> 1..11
<223> /mol_type="protein" /organism="Homo sapiens"

<400> 14

```
Ser Gly Asp Asn Ile Pro Leu Lys Tyr Val Ser
1               5                   10
```

<210> 15
<211> 7
<212> PRT
<213> Homo sapiens

<220>
<221> SOURCE
<222> 1..7
<223> /mol_type="protein" /organism="Homo sapiens"

<400> 15

Asp Asp Asn Lys Arg Pro Ser
1 5

<210> 16
<211> 11
<212> PRT
<213> Homo sapiens

<220>
<221> SOURCE
<222> 1..11
<223> /mol_type="protein" /organism="Homo sapiens"

<400> 16

Ser Ser Trp Asp Thr Leu Asp Ile Phe Asn Val
1 5 10

<210> 17
<211> 122
<212> PRT
<213> Homo sapiens

<220>
<221> SOURCE
<222> 1..122
<223> /mol_type="protein" /organism="Homo sapiens"

<400> 17

Gln Val Glu Leu Val Glu Ser Gly Gly Gly Leu Val Gln Pro Gly Gly
1 5 10 15

Ser Leu Arg Leu Ser Cys Ala Ala Ser Gly Phe Thr Phe Ser Asn Tyr
20 25 30
Trp Met His Trp Val Arg Gln Ala Pro Gly Lys Gly Leu Glu Trp Val
35 40 45
Ser Gly Ile Ser Gly Trp Ser Ser Trp Thr Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Arg Phe Gly Ile Asp Ala Tyr Thr Lys Val Tyr Phe Asp Tyr Trp
100 105 110
Gly Gln Gly Thr Leu Val Thr Val Ser Ser
115 120

<210> 18
<211> 109
<212> PRT
<213> Homo sapiens

<220>
<221> SOURCE
<222> 1..109
<223> /mol_type="protein" /organism="Homo sapiens"

<400> 18

```
Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu
65                  70                  75                  80
Asp Glu Ala Asp Tyr Tyr Cys Ser Ser Trp Asp Thr Leu Asp Ile Phe
                85                  90                  95
Asn Val Phe Gly Gly Gly Thr Lys Leu Thr Val Leu Gly
            100                 105
```

<210> 19
<211> 452
<212> PRT
<213> Homo sapiens

<220>
<221> SOURCE
<222> 1..452
<223> /mol_type="protein" /organism="Homo sapiens"

<400> 19

```
Gln Val Glu Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Trp Met His Trp Val Arg Gln Ala Pro Gly Lys Gly Leu Glu Trp Val
        35                  40                  45
Ser Gly Ile Ser Gly Trp Ser Ser Trp Thr Tyr Tyr Ala Asp Ser Val
    50                  55                  60
Lys Gly Arg Phe Thr Ile Ser Arg Asp Asn Ser Lys Asn Thr Leu Tyr
```

```
65                  70                  75                  80
Leu Gln Met Asn Ser Leu Arg Ala Glu Asp Thr Ala Val Tyr Tyr Cys
                85                  90                  95
Ala Arg Phe Gly Ile Asp Ala Tyr Thr Lys Val Tyr Phe Asp Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450
```

<210> 20
<211> 214
<212> PRT
<213> Homo sapiens

<220>
<221> SOURCE
<222> 1..214
<223> /mol_type="protein" /organism="Homo sapiens"

<400> 20

```
Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu
65                  70                  75                  80
Asp Glu Ala Asp Tyr Tyr Cys Ser Ser Trp Asp Thr Leu Asp Ile Phe
                85                  90                  95
Asn Val Phe Gly Gly Gly Thr Lys Leu Thr Val Leu Gly Gln Pro Lys
            100                 105                 110
Ala Ala Pro Ser Val Thr Leu Phe Pro Pro Ser Ser Glu Glu Leu Gln
        115                 120                 125
Ala Asn Lys Ala Thr Leu Val Cys Leu Ile Ser Asp Phe Tyr Pro Gly
    130                 135                 140
Ala Val Thr Val Ala Trp Lys Gly Asp Ser Ser Pro Val Lys Ala Gly
145                 150                 155                 160
Val Glu Thr Thr Thr Pro Ser Lys Gln Ser Asn Asn Lys Tyr Ala Ala
                165                 170                 175
Ser Ser Tyr Leu Ser Leu Thr Pro Glu Gln Trp Lys Ser His Arg Ser
            180                 185                 190
Tyr Ser Cys Gln Val Thr His Glu Gly Ser Thr Val Glu Lys Thr Val
        195                 200                 205
Ala Pro Thr Glu Cys Ser
    210
```

<210> 21
<211> 5
<212> PRT
<213> Homo sapiens

<220>
<221> SOURCE
<222> 1..5
<223> /mol_type="protein" /organism="Homo sapiens"

<400> 21

```
Asn Tyr Trp Met His
1               5
```

<210> 22
<211> 17
<212> PRT
<213> Homo sapiens

<220>
<221> SOURCE
<222> 1..17
<223> /mol_type="protein" /organism="Homo sapiens"

<400> 22

Gly Ile Ser Gly Trp Ser Ser Trp Thr Tyr Tyr Ala Asp Ser Val Lys
1 5 10 15
Gly

<210> 23
<211> 13
<212> PRT
<213> Homo sapiens

<220>
<221> SOURCE
<222> 1..13
<223> /mol_type="protein" /organism="Homo sapiens"

<400> 23

Phe Gly Ile Asp Ala Tyr Thr Lys Val Tyr Phe Asp Tyr
1 5 10

<210> 24
<211> 11
<212> PRT
<213> Homo sapiens

<220>
<221> SOURCE
<222> 1..11
<223> /mol_type="protein" /organism="Homo sapiens"

<400> 24

Ser Gly Asp Asn Ile Pro Leu Lys Tyr Val Ser
1 5 10

<210> 25
<211> 7
<212> PRT
<213> Homo sapiens

<220>
<221> SOURCE
<222> 1..7
<223> /mol_type="protein" /organism="Homo sapiens"

<400> 25

Asp Asp Asn Lys Arg Pro Ser
1 5

<210> 26
<211> 11
<212> PRT
<213> Homo sapiens

<220>
<221> SOURCE
<222> 1..11

<223> /mol_type="protein" /organism="Homo sapiens"

<400> 26

```
Gln Ser Tyr Asp Tyr Phe Pro Lys Phe Val Val
1               5                   10
```

<210> 27
<211> 122
<212> PRT
<213> Homo sapiens

<220>
<221> SOURCE
<222> 1..122
<223> /mol_type="protein" /organism="Homo sapiens"

<400> 27

```
Gln Val Glu Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Asn Tyr
            20                  25                  30
Trp Met His Trp Val Arg Gln Ala Pro Gly Lys Gly Leu Glu Trp Val
        35                  40                  45
Ser Gly Ile Ser Gly Trp Ser Ser Trp Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Phe Gly Ile Asp Ala Tyr Thr Lys Val Tyr Phe Asp Tyr Trp
            100                 105                 110
Gly Gln Gly Thr Leu Val Thr Val Ser Ser
        115                 120
```

<210> 28
<211> 109
<212> PRT
<213> Homo sapiens

<220>
<221> SOURCE
<222> 1..109
<223> /mol_type="protein" /organism="Homo sapiens"

<400> 28

```
Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu
65                  70                  75                  80
Asp Glu Ala Asp Tyr Tyr Cys Gln Ser Tyr Asp Tyr Phe Pro Lys Phe
                85                  90                  95
Val Val Phe Gly Gly Gly Thr Lys Leu Thr Val Leu Gly
            100                 105
```

<210> 29
<211> 452
<212> PRT
<213> Homo sapiens

<220>
<221> SOURCE
<222> 1..452
<223> /mol_type="protein" /organism="Homo sapiens"

<400> 29

```
Gln Val Glu Leu Val Glu Ser Gly Gly Gly Leu Val Gln Pro Gly Gly
```

```
1               5                   10                  15
Ser Leu Arg Leu Ser Cys Ala Ala Ser Gly Phe Thr Phe Ser Asn Tyr
            20                  25                  30
Trp Met His Trp Val Arg Gln Ala Pro Gly Lys Gly Leu Glu Trp Val
        35                  40                  45
Ser Gly Ile Ser Gly Trp Ser Ser Trp Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Phe Gly Ile Asp Ala Tyr Thr Lys Val Tyr Phe Asp Tyr Trp
            100                 105                 110
Gly Gln Gly Thr Leu Val Thr Val Ser Ser Ala Ser Thr Lys Gly Pro
        115                 120                 125
Ser Val Phe Pro Leu Ala Pro Ser Ser Lys Ser Thr Ser Gly Gly Thr
    130                 135                 140
Ala Ala Leu Gly Cys Leu Val Lys Asp Tyr Phe Pro Glu Pro Val Thr
145                 150                 155                 160
Val Ser Trp Asn Ser Gly Ala Leu Thr Ser Gly Val His Thr Phe Pro
                165                 170                 175
Ala Val Leu Gln Ser Ser Gly Leu Tyr Ser Leu Ser Ser Val Val Thr
            180                 185                 190
Val Pro Ser Ser Ser Leu Gly Thr Gln Thr Tyr Ile Cys Asn Val Asn
        195                 200                 205
His Lys Pro Ser Asn Thr Lys Val Asp Lys Lys Val Glu Pro Lys Ser
    210                 215                 220
Cys Asp Lys Thr His Thr Cys Pro Pro Cys Pro Ala Pro Glu Leu Leu
225                 230                 235                 240
Gly Gly Pro Ser Val Phe Leu Phe Pro Pro Lys Pro Lys Asp Thr Leu
                245                 250                 255
Met Ile Ser Arg Thr Pro Glu Val Thr Cys Val Val Val Asp Val Ser
            260                 265                 270
His Glu Asp Pro Glu Val Lys Phe Asn Trp Tyr Val Asp Gly Val Glu
        275                 280                 285
Val His Asn Ala Lys Thr Lys Pro Arg Glu Glu Gln Tyr Asn Ser Thr
    290                 295                 300
Tyr Arg Val Val Ser Val Leu Thr Val Leu His Gln Asp Trp Leu Asn
305                 310                 315                 320
Gly Lys Glu Tyr Lys Cys Lys Val Ser Asn Lys Ala Leu Pro Ala Pro
                325                 330                 335
Ile Glu Lys Thr Ile Ser Lys Ala Lys Gly Gln Pro Arg Glu Pro Gln
            340                 345                 350
Val Tyr Thr Leu Pro Pro Ser Arg Asp Glu Leu Thr Lys Asn Gln Val
        355                 360                 365
Ser Leu Thr Cys Leu Val Lys Gly Phe Tyr Pro Ser Asp Ile Ala Val
    370                 375                 380
Glu Trp Glu Ser Asn Gly Gln Pro Glu Asn Asn Tyr Lys Thr Thr Pro
385                 390                 395                 400
Pro Val Leu Asp Ser Asp Gly Ser Phe Phe Leu Tyr Ser Lys Leu Thr
                405                 410                 415
Val Asp Lys Ser Arg Trp Gln Gln Gly Asn Val Phe Ser Cys Ser Val
            420                 425                 430
Met His Glu Ala Leu His Asn His Tyr Thr Gln Lys Ser Leu Ser Leu
        435                 440                 445
Ser Pro Gly Lys
    450
```

<210> 30
<211> 214
<212> PRT

<213> Homo sapiens

<220>
<221> SOURCE
<222> 1..214
<223> /mol_type="protein" /organism="Homo sapiens"

<400> 30

```
Asp Ile Glu Leu Thr Gln Pro Pro Ser Val Ser Val Ala Pro Gly Gln
1               5                   10                  15
Thr Ala Arg Ile Ser Cys Ser Gly Asp Asn Ile Pro Leu Lys Tyr Val
            20                  25                  30
Ser Trp Tyr Gln Gln Lys Pro Gly Gln Ala Pro Val Leu Val Ile His
        35                  40                  45
Asp Asp Asn Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Glu
65                  70                  75                  80
Asp Glu Ala Asp Tyr Tyr Cys Gln Ser Tyr Asp Tyr Phe Pro Lys Phe
                85                  90                  95
Val Val Phe Gly Gly Gly Thr Lys Leu Thr Val Leu Gly Gln Pro Lys
            100                 105                 110
Ala Ala Pro Ser Val Thr Leu Phe Pro Pro Ser Ser Glu Glu Leu Gln
        115                 120                 125
Ala Asn Lys Ala Thr Leu Val Cys Leu Ile Ser Asp Phe Tyr Pro Gly
    130                 135                 140
Ala Val Thr Val Ala Trp Lys Gly Asp Ser Ser Pro Val Lys Ala Gly
145                 150                 155                 160
Val Glu Thr Thr Thr Pro Ser Lys Gln Ser Asn Asn Lys Tyr Ala Ala
                165                 170                 175
Ser Ser Tyr Leu Ser Leu Thr Pro Glu Gln Trp Lys Ser His Arg Ser
            180                 185                 190
Tyr Ser Cys Gln Val Thr His Glu Gly Ser Thr Val Glu Lys Thr Val
        195                 200                 205
Ala Pro Thr Glu Cys Ser
    210
```

<210> 31
<211> 5
<212> PRT
<213> Homo sapiens

<220>
<221> SOURCE
<222> 1..5
<223> /mol_type="protein" /organism="Homo sapiens"

<400> 31

```
Ser Tyr Trp Met Ser
1               5
```

<210> 32
<211> 17
<212> PRT
<213> Homo sapiens

<220>

<221> SOURCE
<222> 1..17
<223> /mol_type="protein" /organism="Homo sapiens"

<400> 32

Ser Ile Thr Ser Tyr Gly Ser Phe Thr Tyr Tyr Ala Asp Ser Val Lys
1 5 10 15
Gly

<210> 33
<211> 8
<212> PRT
<213> Homo sapiens

<220>
<221> SOURCE
<222> 1..8
<223> /mol_type="protein" /organism="Homo sapiens"

<400> 33

Asn Met Tyr Thr His Phe Asp Ser
1 5

<210> 34
<211> 13
<212> PRT
<213> Homo sapiens

<220>
<221> SOURCE
<222> 1..13
<223> /mol_type="protein" /organism="Homo sapiens"

<400> 34

Ser Gly Ser Ser Ser Asn Ile Gly Ser Asn Ser Val Ser
1 5 10

<210> 35
<211> 7
<212> PRT
<213> Homo sapiens

<220>
<221> SOURCE
<222> 1..7
<223> /mol_type="protein" /organism="Homo sapiens"

<400> 35

Asp Asn Ser Lys Arg Pro Ser
1 5

<210> 36
<211> 11

<212> PRT
<213> Homo sapiens

<220>
<221> SOURCE
<222> 1..11
<223> /mol_type="protein" /organism="Homo sapiens"

<400> 36

```
Gln Ser Arg Asp Thr Tyr Gly Tyr Tyr Trp Val
1               5                   10
```

<210> 37
<211> 117
<212> PRT
<213> Homo sapiens

<220>
<221> SOURCE
<222> 1..117
<223> /mol_type="protein" /organism="Homo sapiens"

<400> 37

```
Gln Val Glu Leu Val Glu Ser Gly Gly Gly Leu Val Gln Pro Gly Gly
1               5                   10                  15
Ser Leu Arg Leu Ser Cys Ala Ala Ser Gly Phe Thr Phe Thr Ser Tyr
            20                  25                  30
Trp Met Ser Trp Val Arg Gln Ala Pro Gly Lys Gly Leu Glu Leu Val
        35                  40                  45
Ser Ser Ile Thr Ser Tyr Gly Ser Phe Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Asn Met Tyr Thr His Phe Asp Ser Trp Gly Gln Gly Thr Leu
            100                 105                 110
Val Thr Val Ser Ser
        115
```

<210> 38
<211> 111
<212> PRT
<213> Homo sapiens

<220>
<221> SOURCE
<222> 1..111
<223> /mol_type="protein" /organism="Homo sapiens"

<400> 38

```
Asp Ile Val Leu Thr Gln Pro Pro Ser Val Ser Gly Ala Pro Gly Gln
1               5                   10                  15
Arg Val Thr Ile Ser Cys Ser Gly Ser Ser Ser Asn Ile Gly Ser Asn
            20                  25                  30
Ser Val Ser Trp Tyr Gln Gln Leu Pro Gly Thr Ala Pro Lys Leu Leu
        35                  40                  45
Ile Tyr Asp Asn Ser Lys Arg Pro Ser Gly Val Pro Asp Arg Phe Ser
    50                  55                  60
Gly Ser Lys Ser Gly Thr Ser Ala Ser Leu Ala Ile Thr Gly Leu Gln
65                  70                  75                  80
Ser Glu Asp Glu Ala Asp Tyr Tyr Cys Gln Ser Arg Asp Thr Tyr Gly
                85                  90                  95
Tyr Tyr Trp Val Phe Gly Gly Gly Thr Lys Leu Thr Val Leu Gly
            100                 105                 110
```

<210> 39
<211> 447
<212> PRT
<213> Homo sapiens

<220>
<221> SOURCE
<222> 1..447
<223> /mol_type="protein" /organism="Homo sapiens"

<400> 39

Gln Val Glu Leu Val Glu Ser Gly Gly Gly Leu Val Gln Pro Gly Gly
1 5 10 15
Ser Leu Arg Leu Ser Cys Ala Ala Ser Gly Phe Thr Phe Thr Ser Tyr
20 25 30
Trp Met Ser Trp Val Arg Gln Ala Pro Gly Lys Gly Leu Glu Leu Val
35 40 45
Ser Ser Ile Thr Ser Tyr Gly Ser Phe Thr Tyr Tyr Ala Asp Ser Val
50 55 60
Lys Gly Arg Phe Thr Ile Ser Arg Asp Asn Ser Lys Asn Thr Leu Tyr
65 70 75 80
Leu Gln Met Asn Ser Leu Arg Ala Glu Asp Thr Ala Val Tyr Tyr Cys
85 90 95
Ala Arg Asn Met Tyr Thr His Phe Asp Ser Trp Gly Gln Gly Thr Leu
100 105 110
Val Thr Val Ser Ser Ala Ser Thr Lys Gly Pro Ser Val Phe Pro Leu
115 120 125
Ala Pro Ser Ser Lys Ser Thr Ser Gly Gly Thr Ala Ala Leu Gly Cys
130 135 140
Leu Val Lys Asp Tyr Phe Pro Glu Pro Val Thr Val Ser Trp Asn Ser
145 150 155 160
Gly Ala Leu Thr Ser Gly Val His Thr Phe Pro Ala Val Leu Gln Ser
165 170 175
Ser Gly Leu Tyr Ser Leu Ser Ser Val Val Thr Val Pro Ser Ser Ser
180 185 190
Leu Gly Thr Gln Thr Tyr Ile Cys Asn Val Asn His Lys Pro Ser Asn
195 200 205
Thr Lys Val Asp Lys Lys Val Glu Pro Lys Ser Cys Asp Lys Thr His
210 215 220
Thr Cys Pro Pro Cys Pro Ala Pro Glu Leu Leu Gly Gly Pro Ser Val
225 230 235 240
Phe Leu Phe Pro Pro Lys Pro Lys Asp Thr Leu Met Ile Ser Arg Thr
245 250 255
Pro Glu Val Thr Cys Val Val Val Asp Val Ser His Glu Asp Pro Glu
260 265 270
Val Lys Phe Asn Trp Tyr Val Asp Gly Val Glu Val His Asn Ala Lys
275 280 285
Thr Lys Pro Arg Glu Glu Gln Tyr Asn Ser Thr Tyr Arg Val Val Ser
290 295 300
Val Leu Thr Val Leu His Gln Asp Trp Leu Asn Gly Lys Glu Tyr Lys
305 310 315 320
Cys Lys Val Ser Asn Lys Ala Leu Pro Ala Pro Ile Glu Lys Thr Ile
325 330 335
Ser Lys Ala Lys Gly Gln Pro Arg Glu Pro Gln Val Tyr Thr Leu Pro
340 345 350
Pro Ser Arg Asp Glu Leu Thr Lys Asn Gln Val Ser Leu Thr Cys Leu
355 360 365
Val Lys Gly Phe Tyr Pro Ser Asp Ile Ala Val Glu Trp Glu Ser Asn
370 375 380
Gly Gln Pro Glu Asn Asn Tyr Lys Thr Thr Pro Pro Val Leu Asp Ser
385 390 395 400
Asp Gly Ser Phe Phe Leu Tyr Ser Lys Leu Thr Val Asp Lys Ser Arg
405 410 415
Trp Gln Gln Gly Asn Val Phe Ser Cys Ser Val Met His Glu Ala Leu
420 425 430
His Asn His Tyr Thr Gln Lys Ser Leu Ser Leu Ser Pro Gly Lys
435 440 445

<210> 40
<211> 216
<212> PRT
<213> Homo sapiens

<220>
<221> SOURCE
<222> 1..216
<223> /mol_type="protein" /organism="Homo sapiens"

<400> 40

```
Asp Ile Val Leu Thr Gln Pro Pro Ser Val Ser Gly Ala Pro Gly Gln
1               5                   10                  15
Arg Val Thr Ile Ser Cys Ser Gly Ser Ser Ser Asn Ile Gly Ser Asn
            20                  25                  30
Ser Val Ser Trp Tyr Gln Gln Leu Pro Gly Thr Ala Pro Lys Leu Leu
        35                  40                  45
Ile Tyr Asp Asn Ser Lys Arg Pro Ser Gly Val Pro Asp Arg Phe Ser
    50                  55                  60
Gly Ser Lys Ser Gly Thr Ser Ala Ser Leu Ala Ile Thr Gly Leu Gln
65                  70                  75                  80
Ser Glu Asp Glu Ala Asp Tyr Tyr Cys Gln Ser Arg Asp Thr Tyr Gly
                85                  90                  95
Tyr Tyr Trp Val Phe Gly Gly Gly Thr Lys Leu Thr Val Leu Gly Gln
            100                 105                 110
Pro Lys Ala Ala Pro Ser Val Thr Leu Phe Pro Pro Ser Ser Glu Glu
        115                 120                 125
Leu Gln Ala Asn Lys Ala Thr Leu Val Cys Leu Ile Ser Asp Phe Tyr
    130                 135                 140
Pro Gly Ala Val Thr Val Ala Trp Lys Gly Asp Ser Ser Pro Val Lys
145                 150                 155                 160
Ala Gly Val Glu Thr Thr Thr Pro Ser Lys Gln Ser Asn Asn Lys Tyr
                165                 170                 175
Ala Ala Ser Ser Tyr Leu Ser Leu Thr Pro Glu Gln Trp Lys Ser His
            180                 185                 190
Arg Ser Tyr Ser Cys Gln Val Thr His Glu Gly Ser Thr Val Glu Lys
        195                 200                 205
Thr Val Ala Pro Thr Glu Cys Ser
    210                 215
```

<210> 41
<211> 121
<212> PRT
<213> Homo sapiens

<220>
<221> SOURCE
<222> 1..121
<223> /mol_type="protein" /organism="Homo sapiens"

<400> 41

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Asp Ile Asn Trp Val Arg Gln Ala Thr Gly Gln Gly Leu Glu Trp Met
        35                  40                  45
Gly Trp Met Asn Pro Asn Ser Gly Asn Thr Gly Tyr Ala Gln Lys Phe
    50                  55                  60
Gln Gly Arg Val Thr Met Thr Arg Asp Thr Ser Ile Ser Thr Ala Tyr
```

```
65                  70                  75                  80
Met Glu Leu Ser Ser Leu Arg Ser Glu Asp Thr Ala Val Tyr Tyr Cys
                85                  90                  95
Ala Arg Asp Pro Tyr Tyr Tyr Tyr Tyr Gly Met Asp Val Trp Gly Gln
            100                 105                 110
Gly Thr Thr Val Thr Val Ser Ser Ala
        115                 120
```

<210> 42
<211> 112
<212> PRT
<213> Homo sapiens

<220>
<221> SOURCE
<222> 1..112
<223> /mol_type="protein" /organism="Homo sapiens"

<400> 42

```
Gln Ser Val Leu Thr Gln Pro Pro Ser Val Ser Ala Ala Pro Gly Gln
1               5                   10                  15
Lys Val Thr Ile Ser Cys Ser Gly Ser Ser Ser Asn Ile Glu Asn Asn
            20                  25                  30
His Val Ser Trp Tyr Gln Gln Leu Pro Gly Thr Ala Pro Lys Leu Leu
        35                  40                  45
Ile Tyr Asp Asn Asn Lys Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Asp Tyr Tyr Cys Glu Thr Trp Asp Thr Ser Leu
                85                  90                  95
Ser Ala Gly Arg Val Phe Gly Gly Gly Thr Lys Leu Thr Val Leu Gly
            100                 105                 110
```

<210> 43
<211> 121
<212> PRT
<213> Homo sapiens

<220>
<221> SOURCE
<222> 1..121
<223> /mol_type="protein" /organism="Homo sapiens"

<400> 43

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Asp Ile Asn Trp Val Arg Gln Ala Thr Gly Gln Gly Leu Glu Trp Met
        35                  40                  45
Gly Trp Met Asn Pro Asn Ser Gly Asn Thr Gly Tyr Ala Gln Lys Phe
    50                  55                  60
Gln Gly Arg Val Thr Met Thr Arg Asn Thr Ser Ile Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Asp Pro Tyr Tyr Tyr Tyr Tyr Gly Met Asp Val Trp Gly Gln
            100                 105                 110
Gly Thr Thr Val Thr Val Ser Ser Ala
        115                 120
```

<210> 44
<211> 112
<212> PRT
<213> Homo sapiens

<220>
<221> SOURCE
<222> 1..112
<223> /mol_type="protein" /organism="Homo sapiens"

<400> 44

```
Gln Ser Val Leu Thr Gln Pro Pro Ser Val Ser Ala Ala Pro Gly Gln
1               5                   10                  15
Lys Val Thr Ile Ser Cys Ser Gly Ser Ser Ser Asn Ile Glu Asn Asn
            20                  25                  30
His Val Ser Trp Tyr Gln Gln Leu Pro Gly Thr Ala Pro Lys Leu Leu
        35                  40                  45
Ile Tyr Asp Asn Asn Lys Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Asp Tyr Tyr Cys Glu Thr Trp Asp Thr Ser Leu
                85                  90                  95
Ser Ala Gly Arg Val Phe Gly Gly Gly Thr Lys Leu Thr Val Leu Gly
            100                 105                 110
```

<210> 45
<211> 450
<212> PRT
<213> Homo sapiens

<220>
<221> SOURCE
<222> 1..450
<223> /mol_type="protein" /organism="Homo sapiens"

<400> 45

```
Glu Val Gln Leu Leu Glu Ser Gly Gly Gly Leu Val Gln Pro Gly Gly
1               5                   10                  15
Ser Leu Arg Leu Ser Cys Thr Ala Ser Gly Phe Thr Phe Ser Ser Tyr
            20                  25                  30
Ala Met Asn Trp Val Arg Gln Ala Pro Gly Lys Gly Leu Glu Trp Val
        35                  40                  45
Ser Ala Ile Ser Gly Ser Gly Gly Thr Thr Phe Tyr Ala Asp Ser Val
    50                  55                  60
Lys Gly Arg Phe Thr Ile Ser Arg Asp Asn Ser Arg Thr Thr Leu Tyr
65                  70                  75                  80
Leu Gln Met Asn Ser Leu Arg Ala Glu Asp Thr Ala Val Tyr Tyr Cys
                85                  90                  95
Ala Lys Asp Leu Gly Trp Ser Asp Ser Tyr Tyr Tyr Tyr Tyr Gly Met
            100                 105                 110
Asp Val Trp Gly Gln Gly Thr Thr Val Thr Val Ser Ser Ala Ser Thr
        115                 120                 125
Lys Gly Pro Ser Val Phe Pro Leu Ala Pro Cys Ser Arg Ser Thr Ser
    130                 135                 140
Glu Ser Thr Ala Ala Leu Gly Cys Leu Val Lys Asp Tyr Phe Pro Glu
145                 150                 155                 160
Pro Val Thr Val Ser Trp Asn Ser Gly Ala Leu Thr Ser Gly Val His
                165                 170                 175
Thr Phe Pro Ala Val Leu Gln Ser Ser Gly Leu Tyr Ser Leu Ser Ser


            180                 185                 190
Val Val Thr Val Pro Ser Ser Asn Phe Gly Thr Gln Thr Tyr Thr Cys
        195                 200                 205
Asn Val Asp His Lys Pro Ser Asn Thr Lys Val Asp Lys Thr Val Glu
    210                 215                 220
Arg Lys Cys Cys Val Glu Cys Pro Pro Cys Pro Ala Pro Pro Val Ala
225                 230                 235                 240
Gly Pro Ser Val Phe Leu Phe Pro Pro Lys Pro Lys Asp Thr Leu Met
                245                 250                 255
Ile Ser Arg Thr Pro Glu Val Thr Cys Val Val Val Asp Val Ser His
            260                 265                 270
Glu Asp Pro Glu Val Gln Phe Asn Trp Tyr Val Asp Gly Val Glu Val
        275                 280                 285
His Asn Ala Lys Thr Lys Pro Arg Glu Glu Gln Phe Asn Ser Thr Phe
    290                 295                 300
Arg Val Val Ser Val Leu Thr Val Val His Gln Asp Trp Leu Asn Gly
305                 310                 315                 320
Lys Glu Tyr Lys Cys Lys Val Ser Asn Lys Gly Leu Pro Ala Pro Ile
                325                 330                 335
Glu Lys Thr Ile Ser Lys Thr Lys Gly Gln Pro Arg Glu Pro Gln Val
            340                 345                 350
Tyr Thr Leu Pro Pro Ser Arg Glu Glu Met Thr Lys Asn Gln Val Ser
        355                 360                 365
Leu Thr Cys Leu Val Lys Gly Phe Tyr Pro Ser Asp Ile Ala Val Glu
    370                 375                 380
Trp Glu Ser Asn Gly Gln Pro Glu Asn Asn Tyr Lys Thr Thr Pro Pro
385                 390                 395                 400
Met Leu Asp Ser Asp Gly Ser Phe Phe Leu Tyr Ser Lys Leu Thr Val
                405                 410                 415
Asp Lys Ser Arg Trp Gln Gln Gly Asn Val Phe Ser Cys Ser Val Met
            420                 425                 430
His Glu Ala Leu His Asn His Tyr Thr Gln Lys Ser Leu Ser Leu Ser
        435                 440                 445
Pro Gly
    450
```

<210> 46

<211> 214
<212> PRT
<213> Homo sapiens

<220>
<221> SOURCE
<222> 1..214
<223> /mol_type="protein" /organism="Homo sapiens"

<400> 46

```
Asp Ile Gln Met Thr Gln Phe Pro Ser Ser Leu Ser Ala Ser Val Gly
1               5                   10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Arg Leu His Arg Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro Cys
                85                  90                  95
Ser Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly


        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```